# EUROPEAN PATENT APPLICATION

(11) **EP 4 111 987 A1**
(43) Date of publication of application: **04.01.2023**
(21) Application number: 21775649.3
(22) Date of filing: 08.03.2021
(51) Int. Cl.: A61B 17/135

(54) **COMPRESSION DEVICE AND METHOD FOR AFFIXING COMPRESSION DEVICE**

(30) Priority: 27.03.2020 JP 2020059022
(71) Applicant: TERUMO Kabushiki Kaisha, Tokyo 151-0072 (JP)
(72) Inventor: KAWAURA, Masakatsu, Campbell, California 95008 (US)
(74) Representative: Casalonga
(86) International application number: PCT/JP2021/009088
(87) International publication number: WO 2021/192974

(57) **Abstract**

[Problem] Provided is a compression device that is capable of being easily positioned at an appropriate position on a biological surface and a method for adhering the compression device.

[Means for Resolution] The compression device according to this disclosure includes: an adhesive sheet having an adhesion surface; and a compression member. The compression member includes: a pressing body configured to press a biological surface; and a holding body that is fixed to the adhesive sheet and that is configured to hold the pressing body to be extendable. The pressing body includes: an inflatable portion that is inflatable in the thickness direction of the adhesive sheet; and an extending portion that extends from the inflatable portion and that is wound around the holding body to extend from the inflatable portion to an upper surface side of the holding body on a side opposite to the inflatable portion with the holding body interposed therebetween. The extending portion includes a light-transmitting portion having translucency in the thickness direction at a position at which an upper surface of the holding body is covered. The holding body includes, at a position overlapping with the light-transmitting portion of the extending portion in a plan view seen in the thickness direction, a marker portion configured to be visually identified.

## Description

### Technical Field

This disclosure generally relates to a compression device and a method for adhering a compression device.

### Background Art

In recent years, various forms of examinations and treatments using catheters have been performed in medical institutions. A catheter is percutaneously inserted into a blood vessel from a puncture site formed at a wrist, an inguinal region, and the like, and is carried through the blood vessel to a site to be examined or treated, for example. After an examination or treatment by a health care worker is completed, an elongated insertion member such as a puncture needle, a catheter, and a sheath used for introducing a catheter into a living body is removed from a puncture site, and the puncture site is stopped from bleeding.

Patent document 1 discloses a dressing as a compression device that applies compression to a wound of a patient after removing a sheath. The dressing disclosed in the patent document 1 includes an inflatable bladder having a deflated state in which a membrane is adjacent to an end wall and an inflated state in which the membrane is spaced from the end wall. In addition, the dressing disclosed in the patent document 1 includes a holding portion that holds the bladder against a skin of the patient at a position at which the wound is substantially covered. In the patent document 1, the holding portion includes a flexible web that is connected to the end wall of the bladder and that protrudes outward from the end wall of the bladder, and one surface of the flexible web is provided with an adhesive layer that is adhered to the skin of the patient.

### Citation List

### Patent Literature

Patent document 1: JP-T-2005-521464

### Summary of Invention

### Technical Problem

In the dressing as the compression device described in the patent document 1, the adhesive layer provided on the one surface of the flexible web is adhered to the skin of the patient that is a biological surface, and the bladder is brought into the inflated state, so that the wound of the patient can be compressed by the bladder.

However, in the dressing described in the patent document 1, there is still room for improvement in efficiency of aligning the bladder to a predetermined compression position on the biological surface.

An object of this disclosure is to provide a compression device that is capable of being easily positioned at an appropriate position on a biological surface and a method for adhering a compression device.

### Solution to Problem

According to a first aspect of this disclosure, a compression device includes: an adhesive sheet having an adhesion surface configured to be adhered to a biological surface; and a compression member that is fixed to the adhesive sheet and that is configured to compress the biological surface. The compression member includes: a pressing body configured to press the biological surface by extending in a thickness direction of the adhesive sheet; and a holding body that is fixed to the adhesive sheet on a side opposite to the adhesion surface and that is configured to hold the pressing body to be extendable in the thickness direction. The pressing body includes: an inflatable portion that is disposed between the biological surface and the holding body in a state in which the adhesion surface of the adhesive sheet is adhered to the biological surface and that is inflatable in the thickness direction; and a flexible extending portion that extends from the inflatable portion, that is wound around the holding body to extend from the inflatable portion to an upper surface side of the holding body on a side opposite to the inflatable portion with the holding body interposed therebetween, and that is locked to the holding body on the upper surface side of the holding body. The extending portion includes a light-transmitting portion having translucency in the thickness direction at a position at which an upper surface of the holding body is covered. The holding body includes, at a position overlapping with the light-transmitting portion of the extending portion in a plan view seen in the thickness direction, a marker portion configured to be visually identified.

According to one embodiment of this disclosure, the holding body defines a through hole penetrating in the thickness direction, and the extending portion is wound around the holding body through the through hole.

According to one embodiment of this disclosure, in the plan view seen in the thickness direction, a receiving portion, which is a region in which the adhesive sheet is not disposed or a region defined by a concave portion in an outer edge of the adhesive sheet and which is configured to receive a medical tube member, is provided outside an outer edge of the holding body, the extending portion is wound around the holding body on a receiving portion side with respect to the inflatable portion, and the inflatable portion is inflated while pivoting about a portion connected to the extending portion as a hinge portion.

According to one embodiment of this disclosure, the marker portion includes a region marker that defines a predetermined region in the plan view seen in the thickness direction.

According to one embodiment of this disclosure, the region marker is a frame line marker that surrounds the predetermined region.

According to one embodiment of this disclosure, the holding body has translucency in the thickness direction in the predetermined region defined by the region marker.

According to one embodiment of this disclosure, the marker portion includes a position marker indicating a predetermined reference position in the predetermined region defined by the region marker in the plan view seen in the thickness direction.

According to one embodiment of this disclosure, the position marker is a line segment marker extending in an arc shape in the predetermined region defined by the region marker in the plan view seen in the thickness direction.

According to a second aspect of this disclosure, there is provided a method for adhering a compression device to a biological surface. The compression device includes: an adhesive sheet having an adhesion surface configured to be adhered to the biological surface; and a compression member that is fixed to the adhesive sheet and that is configured to compress the biological surface. The compression member includes: a pressing body configured to press the biological surface by extending in a thickness direction of the adhesive sheet; and a holding body that is fixed to the adhesive sheet on a side opposite to the adhesion surface and that holds the pressing body to be extendable in the thickness direction. The pressing body includes: an inflatable portion that is disposed between the biological surface and the holding body whenthe adhesion surface of the adhesive sheet is adhered to the biological surface and that is inflatable in the thickness direction; and a flexible extending portion that extends from the inflatable portion, that is wound around the holding body to extend from the inflatable portion to an upper surface side of the holding body that is on a side of the holding body opposite to the inflatable portion with the holding body interposed therebetween, and that is locked to the holding body on the upper surface side of the holding body. The extending portion includes a light-transmitting portion that is translucent in the thickness direction at a position at which an upper surface of the holding body is covered. The holding body includes, at a position overlapping with the light-transmitting portion of the extending portion in a plan view seen in the thickness direction, a marker portion configured to be visually identified. The adhesive sheet is adhered to the biological surface in a state in which an insertion portion of a medical tube member inserted into a living body from the biological surface, the insertion portion that is located on a surface that is the same as the biological surface, is aligned with the marker portion.

According to one embodiment of this disclosure, the marker portion includes a region marker that defines a predetermined region in the plan view seen in the thickness direction, and the adhesive sheet is adhered to the biological surface in a state in which the whole insertion portion of the medical tube member inserted into the living body from the biological surface, the whole insertion portion being located on the same surface as the biological surface, is aligned with the marker portion.

According to one embodiment of this disclosure, the marker portion includes a position marker indicating a predetermined reference position in the predetermined region defined by the region marker in the plan view seen in the thickness direction, and the adhesive sheet is adhered to the biological surface in a state in which the whole insertion portion of the medical tube member inserted into the living body from the biological surface, the whole insertion portion being located on the same surface as the biological surface, is aligned within the region marker, and a state in which a part of the insertion portion is aligned with the position marker.

### Advantageous Effects of Invention

According to this disclosure, the compression device that is capable of being easily positioned at an appropriate position on the biological surface and the method for adhering the compression device can be provided.

### Brief Description of Drawings

[Fig. 1] Fig. 1 is a perspective view of a compression device according to a first embodiment of this disclosure as seen from an upper surface side.
[Fig. 2] Fig. 2 is a top view of the compression device shown in Fig. 1.
[Fig. 3] Fig. 3 is a bottom view of the compression device shown in Fig. 1.
[Fig. 4] Fig. 4 is an exploded perspective view of the compression device shown in Fig. 1.
[Fig. 5] Fig. 5 is a section view of the compression device shown in Fig. 1 in a state in which an inflatable portion is in a deflated form.
[Fig. 6] Fig. 6 is a section view of the compression device shown in Fig. 1 in a state in which the inflatable portion is in an inflated form.
[Fig. 7] Fig. 7 is a flowchart showing an example of a method for compressing a biological surface using the compression device shown in Fig. 1, the method including a method for adhering a compression device, according to the first embodiment of this disclosure.
[Fig. 8A] Fig. 8A is a diagram showing an outline of an adhering step in Fig. 7.
[Fig. 8B] Fig. 8B is a diagram showing an outline of the adhering step in Fig. 7.
[Fig. 8C] Fig. 8C is a diagram showing an outline of a first compression step in Fig. 7.
[Fig. 8D] Fig. 8D is a diagram showing an outline of a removing step in Fig. 7.
[Fig. 8E] Fig. 8E is a diagram showing an outline of a second compression step in Fig. 7.
[Fig. 9A] Fig. 9A is a top view of a holding body of a compression device according to a second embodiment of this disclosure.
[Fig. 9B] Fig. 9B is a top view of a holding body of a compression device according to a third embodiment of this disclosure.
[Fig. 10] Fig. 10 is a bottom view of a compression device according to a fourth embodiment of this disclosure in a state in which a pressing body is removed.
[Fig. 11] Fig. 11 is a schematic diagram illustrating an effect of a marker portion provided on a lower surface of a holding body in the compression device according to an embodiment of this disclosure.
[Fig. 12A] Fig. 12A is a diagram showing a state in which a medical insertion member is inserted into a femoral vein from a biological surface through a connective tissue.
[Fig. 12B] Fig. 12B is a diagram showing a state after the medical insertion member is removed from the state shown in Fig. 12A.
[Fig. 13] Fig. 13 is a diagram showing a state in which perforations shown in Fig. 12B are narrowed or obstructed by the compression device shown in Fig. 1.
[Fig. 14] Fig. 14 is a front view of the state shown in Fig. 13 as seen from a biological surface side.

### Description of Embodiments

Hereinafter, embodiments of a compression device and a method for adhering a compression device according to this disclosure will be exemplified with reference to the drawings. In the drawings, common members and portions are denoted by the same reference numerals.

### [First Embodiment]

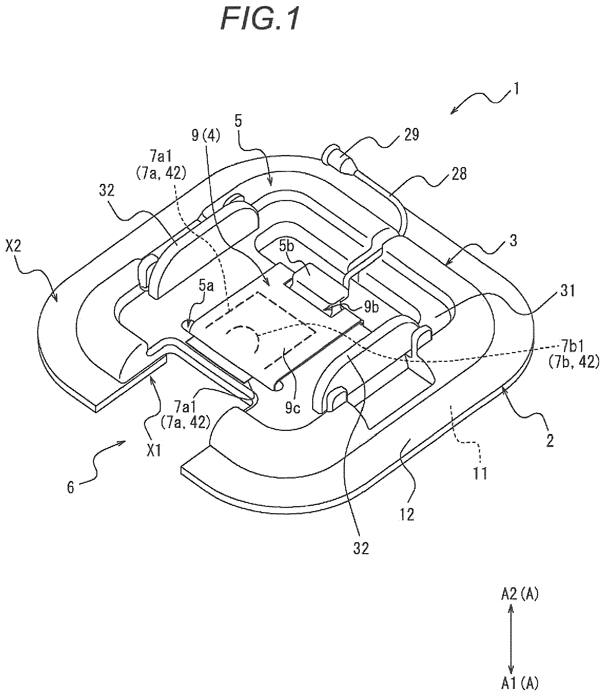

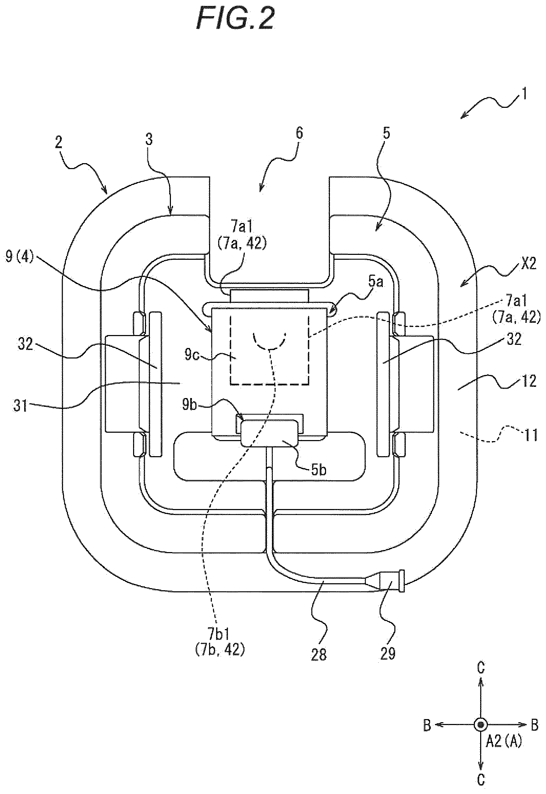

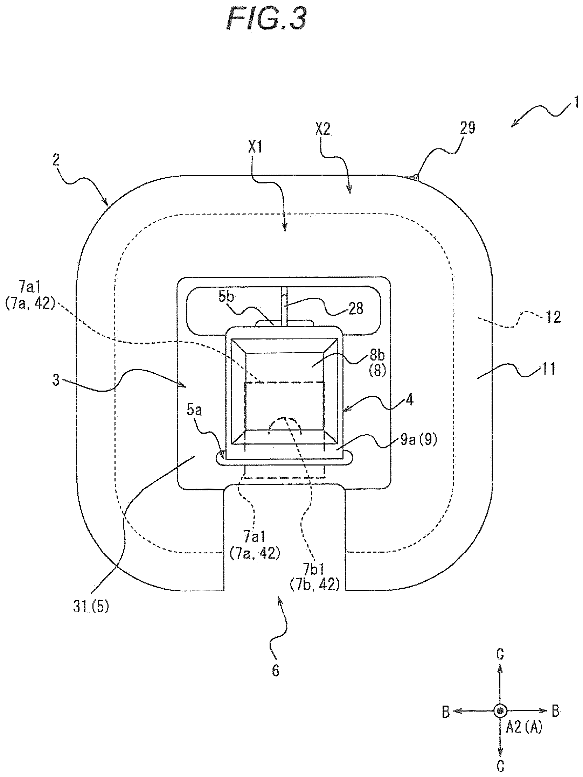

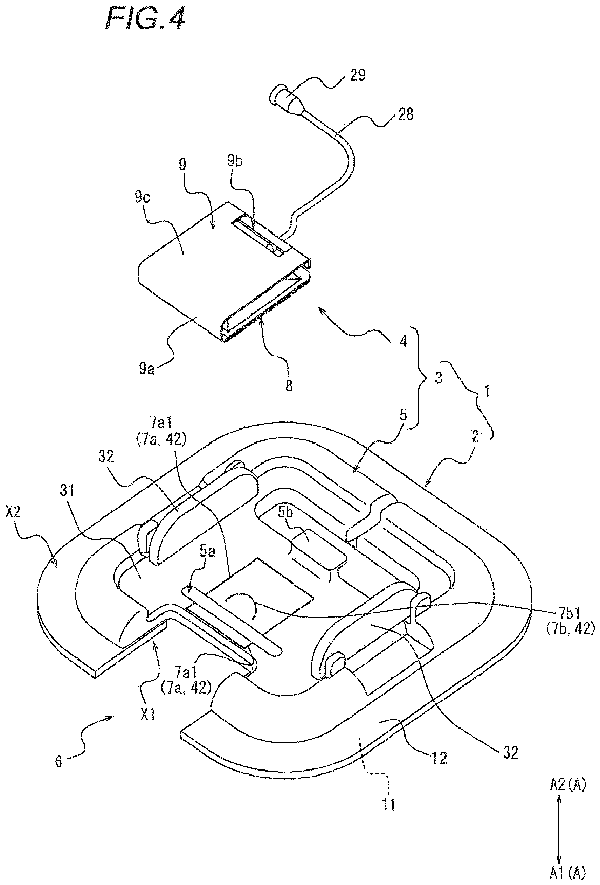

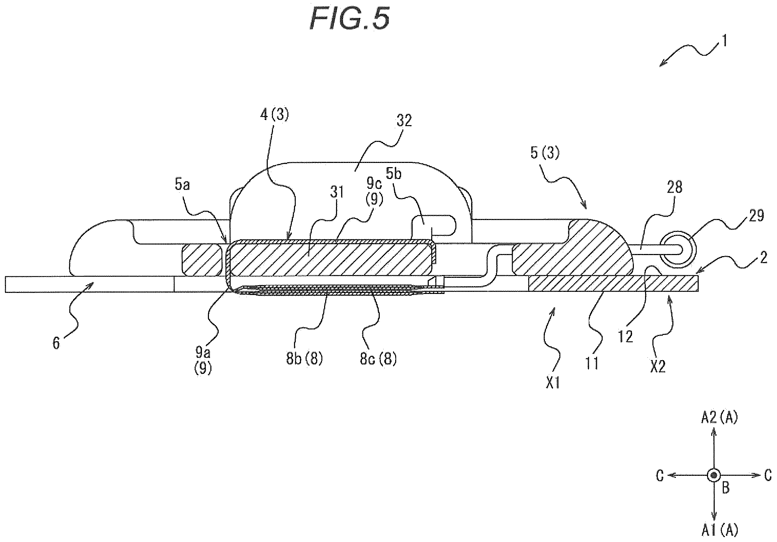

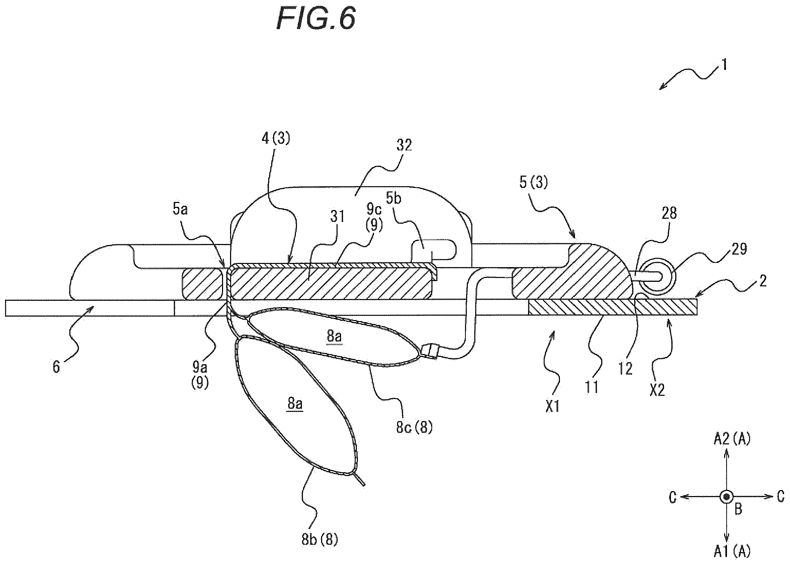

Figs. 1 to 6 are views showing a compression device 1 according to an embodiment of this disclosure. Specifically, Fig. 1 is a perspective view of the compression device 1 as seen from an upper surface side. Figs. 2 and 3 are plan views of the compression device 1. Specifically, Fig. 2 is a top view of the compression device 1. Fig. 3 is a bottom view of the compression device 1. Fig. 4 is an exploded perspective view of the compression device 1. Figs. 5 and 6 are section views of the compression device 1 in the same cross section. The details will be described later, and Figs. 5 and 6 show different states of the compression device 1.

The compression device 1 includes an adhesive sheet 2 and a compression member 3.

The adhesive sheet 2 has an adhesion surface 11, which can be adhered to a biological surface, on one side in a thickness direction A. The compression member 3 according to the present embodiment is attached to an attachment surface 12 on a side opposite to the adhesion surface 11 of the adhesive sheet 2.

Hereinafter, a surface of the adhesive sheet 2 on which the adhesion surface 11 is provided is referred to as a "lower surface of the adhesive sheet 2". In addition, a surface of the adhesive sheet 2 on which the attachment surface 12 is provided is referred to as an "upper surface of the adhesive sheet 2". Hereinafter, for convenience of description, the one side in the thickness direction A, which is a direction from the attachment surface 12 to the adhesion surface 11 in the thickness direction A, may be simply referred to as a "downward direction A1" or a "lower side". In addition, for convenience of description, the other side in the thickness direction A, which is a direction from the adhesion surface 11 to the attachment surface 12 in the thickness direction A, may be simply referred to as an "upward direction A2" or an "upper side". Further, in a plan view (see Figs. 2 and 3) of the compression device 1 seen along the thickness direction A of the adhesive sheet 2, a plan view (see Fig. 2) of the compression device 1 seen from an attachment surface 12 side of the adhesive sheet 2 is simply referred to as a "top view" for convenience of description. In addition, in the plan view (see Figs. 2 and 3) of the compression device 1 seen along the thickness direction A of the adhesive sheet 2, a plan view (see Fig. 3) of the compression device 1 seen from an adhesion surface 11 side of the adhesive sheet 2 is simply referred to as a "bottom view" for convenience of description. In addition, when the top view and the bottom view are not distinguished from each other, the "plan view" may be simply referred to. In addition, unless otherwise specified, the simple descriptions of the "plan view", the "top view", and the "bottom view" mean a plan view, a top view, and a bottom view when an inflatable portion 8 of a pressing body 4, which will be described later, of the compression member 3 is in a deflated form.

The compression member 3 is attached to the adhesive sheet 2. Specifically, the compression member 3 is fixed to the adhesive sheet 2 on a side opposite to the adhesion surface 11. More specifically, the compression member 3 according to the present embodiment is fixed to the attachment surface 12 of the adhesive sheet 2. The compression member 3 can compress the biological surface in a state in which the adhesion surface 11 is adhered to the biological surface. Accordingly, the compression device 1 is fixed to a position on the biological surface by adhering the adhesion surface 11 to the biological surface. According to the compression device 1, a predetermined site on the biological surface can be compressed by the compression member 3 in a state in which the adhesion surface 11 of the adhesive sheet 2 is adhered to the biological surface. The predetermined site on the biological surface includes, for example, a wound on the biological surface or its vicinity formed by inserting a medical insertion member such as a puncture needle, a catheter, and a sheath into a blood vessel of a living body. After the medical insertion member is removed from the living body, bleeding can be stopped by compressing the wound on the biological surface or its vicinity with the compression member 3 for predetermined time.

More specifically, the compression member 3 includes the pressing body 4 and a holding body 5. The pressing body 4 can press the biological surface by extending in the thickness direction A of the adhesive sheet 2. The holding body 5 is fixed to the adhesive sheet 2 on the side opposite to the adhesion surface 11, and holds the pressing body 4 to be extendable in the thickness direction A.

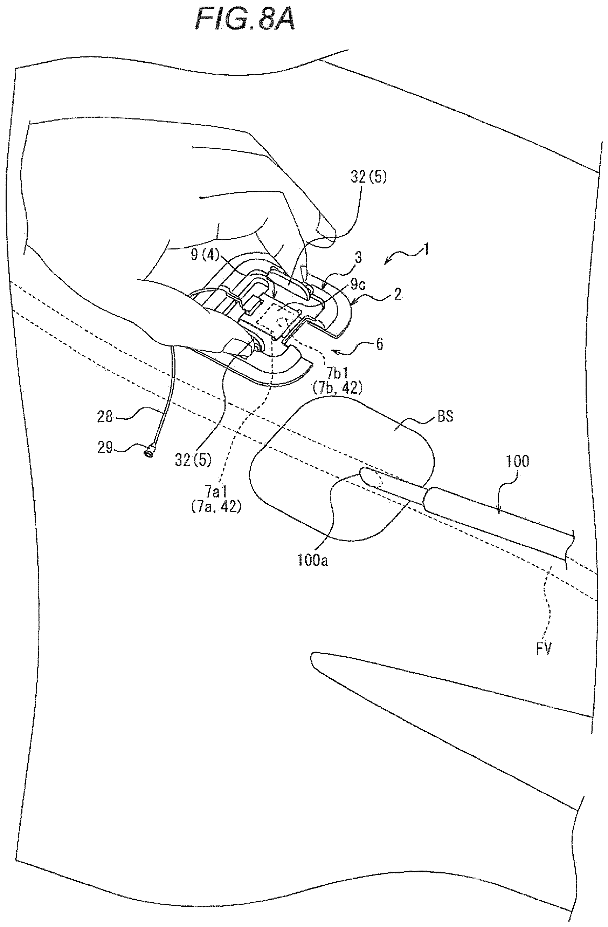

In the plan view of the compression device 1 seen in the thickness direction A (see Figs. 2 and 3), a receiving portion 6, which is a region in which the adhesive sheet 2 is not disposed and which can receive a medical insertion member 100 to be described later (see Fig. 8A or the like), is provided outside an outer edge of the holding body 5 according to the present embodiment. More specifically, the adhesive sheet 2 according to the present embodiment includes a first portion X1 that overlaps with the holding body 5 and a second portion X2 that does not overlap with the holding body 5 in the plan view of the compression device 1 seen in the thickness direction A (see Figs. 2 and 3). As shown in Fig. 2, in the top view of the compression device 1, most of an outer periphery of the outer edge of the holding body 5 according to the present embodiment is surrounded by the second portion X2 of the adhesive sheet 2. In other words, the outer edge of the holding body 5 of the compression device 1 according to the present embodiment includes only a small part on the outside thereof at which the second portion X2 of the adhesive sheet 2 is not disposed. The receiving portion 6 according to the present embodiment is a region around the holding body 5 in which the second portion X2 of the adhesive sheet 2 is not disposed.

The receiving portion 6 according to the present embodiment is a region in which the adhesive sheet 2 is not disposed outside the outer edge of the holding body 5 in the plan view of the compression device 1 seen in the thickness direction A (see Figs. 2 and 3), but is not limited to the configuration. The receiving portion 6 may be a region defined by a concave portion in an outer edge of the adhesive sheet 2. For example, in the plan view of the compression device 1 seen in the thickness direction A (see Figs. 2 and 3), the concave portion recessed from the periphery of the adhesive sheet 2 may be formed in a part of the outer edge of the adhesive sheet 2 outside the outer edge of the holding body 5. In such a case, the receiving portion 6 may be a region defined by the concave portion. That is, the receiving portion 6 may be formed in a region in which the adhesive sheet 2 is disposed outside the outer edge of the holding body 5 in the plan view of the compression device 1 seen in the thickness direction A (see Figs. 2 and 3). In other words, a position in which the receiving portion 6 is formed is not limited to the region in which the adhesive sheet 2 is not disposed outside the outer edge of the holding body 5 in the plan view of the compression device 1 seen in the thickness direction A (see Figs. 2 and 3).

The compression device 1 may not include the receiving portion 6. However, by providing the receiving portion 6, the compression device 1 can be attached to the biological surface in a state in which a portion of the medical insertion member 100 (see Fig. 8A or the like) extending outside the living body passes through the receiving portion 6. Accordingly, the compression device 1 can be easily attached to a desired position on the biological surface even when there is the portion of the medical insertion member 100 (see Fig. 8A or the like) extending outside the living body.

The pressing body 4 includes the inflatable portion 8 and an extending portion 9.

The inflatable portion 8 is disposed between the biological surface and the holding body 5 in a state in which the adhesion surface 11 of the adhesive sheet 2 is adhered to the biological surface (hereinafter, referred to as an "adhering state of the compression device 1 "). The inflatable portion 8 can be inflated in the thickness direction A in the adhering state of the compression device 1. That is, the pressing body 4 according to the present embodiment extends in the thickness direction A by inflating the inflatable portion 8 in the thickness direction A. When the inflatable portion 8 is inflated in the adhering state of the compression device 1, the inflatable portion 8 presses the biological surface by receiving a reaction force from the holding body 5. Hereinafter, a form before the inflatable portion 8 is inflated will be referred to as a "deflated form" of the inflatable portion 8. In addition, a form in which the inflatable portion 8 is inflated from the deflated form is referred to as an "inflated form" of the inflatable portion 8. The compression device 1 according to the present embodiment, in the adhering state, does not compress the biological surface by the inflatable portion 8 when the inflatable portion 8 is in the deflated form. On the other hand, the compression device 1 according to the present embodiment, in the adhering state, compresses the biological surface by the inflatable portion 8 when the inflatable portion 8 is in the inflated form. Fig. 5 shows the deflated form of the inflatable portion 8. Fig. 6 shows the inflated form of the inflatable portion 8.

The extending portion 9 is extended from the inflatable portion 8. In addition, the extending portion 9 has flexibility. The extending portion 9 is wound around the holding body 5. Accordingly, the extending portion 9 extends from the inflatable portion 8 to an upper surface side of the holding body 5 on a side opposite to the inflatable portion 8 with the holding body 5 interposed therebetween. The extending portion 9 is locked to the holding body 5 on the upper surface side of the holding body 5.

The extending portion 9 according to the present embodiment includes a light-transmitting portion 9c having translucency in the thickness direction A at a position at which an upper surface of the holding body 5 is covered. Specifically, an entire portion of the extending portion 9 according to the present embodiment with which the upper surface of the holding body 5 is covered is the light-transmitting portion 9c. More specifically, the extending portion 9 according to the present embodiment has a sheet shape, and has translucency in a thickness direction at any position. Alternatively, the extending portion 9 may include the light-transmitting portion 9c having translucency in the thickness direction in, for example, only a part at the position at which the upper surface of the holding body 5 is covered.

The holding body 5 includes, at a position overlapping with the light-transmitting portion 9c of the extending portion 9 of the pressing body 4 in the plan view seen in the thickness direction A, a marker portion 42 that can be visually identified. More specifically, the holding body 5 according to the present embodiment includes, at a position overlapping with not only the light-transmitting portion 9c of the extending portion 9 of the pressing body 4 but also the inflatable portion 8 of the pressing body 4 in the plan view seen in the thickness direction A, the marker portion 42 that can be visually identified. A configuration of the marker portion 42 is not particularly limited as long as the marker portion 42 can be visually identified at the position overlapping with the light-transmitting portion 9c of the extending portion 9 of the pressing body 4 in the plan view. Therefore, the marker portion 42 may not be provided at the position overlapping with the inflatable portion 8 of the pressing body 4 in the plan view.

The details will be described later, and the marker portion 42 is used for alignment when the compression device 1 is attached to the biological surface. The marker portion 42 according to the present embodiment includes both a region marker 7a and a position marker 7b. The region marker 7a according to the present embodiment indicates a region in which the inflatable portion 8 can compress the biological surface with a compression force of a first predetermined value or more in a state in which the compression device 1 is attached to the biological surface. The position marker 7b according to the present embodiment indicates a reference position for aligning the medical insertion member 100 (see Fig. 8A) requiring a particularly large compression force in a predetermined region that can be identified by the region marker 7a. That is, the position marker 7b according to the present embodiment indicates a position at which the inflatable portion 8 can compress the biological surface with a compression force of a second predetermined value, which is larger than the first predetermined value, or more in the state in which the compression device 1 is attached to the biological surface. That is, the region marker 7a indicates the region that can be compressed with the compression force of the first predetermined value or more, and the position marker 7b indicates the position that can be compressed with the compression force of the second predetermined value larger than the first predetermined value. Therefore, according to the present embodiment, for example, the medical insertion member 100 (see Fig. 8A) requiring a large compression force, such as a large-diameter sheath, is preferably aligned using the position marker 7b in the region marker 7a. The compression force of the first predetermined value, the second predetermined value, or the like means a force of a value measured when the compression device 1 is used under a predetermined use condition in a state in which the compression device 1 is appropriately attached to the biological surface. The "predetermined use condition" means a condition determined by a person who manufactures or sells the compression device 1, such as a fluid amount for inflating the inflatable portion 8, which will be described later, of the compression device 1.

The marker portion 42 may be, for example, one convex portion protruding from the upper surface of the holding body 5 or a concave-convex portion formed by a set of a plurality of convex portions. The marker portion 42 may be, for example, a concave portion such as a groove portion formed on the upper surface of the holding body 5. In addition, the marker portion 42 may be, for example, one convex portion protruding from a lower surface of a portion having translucency of the holding body 5 or a concave-convex portion formed by a set of a plurality of convex portions. The marker portion 42 may be, for example, a concave portion such as a groove portion formed on the lower surface of the portion having translucency of the holding body 5. Further, for example, the marker portion 42 may have a transmittance different from that of the periphery. As an example, the marker portion 42 may be a portion having a transmittance lower than that of the periphery, which is located in the portion having translucency of the holding body 5. In addition, for example, the marker portion 42 may have a color different from that of the periphery. The marker portion 42 may be, for example, a paint or the like with which a part of the upper surface or the lower surface of the holding body 5 is coated. As described above, the configuration of the marker portion 42 is not particularly limited as long as the marker portion 42 can be identified separately from the periphery through the light-transmitting portion 9c of the extending portion 9 of the pressing body 4 in a plan view under a usage environment, particularly a top view (see Fig. 2). The details of the marker portion 42 according to the present embodiment will be described later.

Since the holding body 5 includes, at the position overlapping with the light-transmitting portion 9c in the plan view, the marker portion 42 that can be visually identified, even when the pressing body 4 including the extending portion 9 wound around the holding body 5 is used, a health care worker who operates the compression device 1 can visually identify the marker portion 42 through the light-transmitting portion 9c of the extending portion 9. Therefore, even when the pressing body 4 including the extending portion 9 wound around the holding body 5 is used, the health care worker can align the compression device 1 at an appropriate position on the biological surface using the marker portion 42.

Hereinafter, details of each member and each portion of the compression device 1 according to the present embodiment will be described.

### <Adhesive Sheet 2>

As described above, the adhesive sheet 2 includes the adhesion surface 11 on the one side in the thickness direction A. In addition, as described above, the adhesive sheet 2 includes the attachment surface 12 on the other side in the thickness direction A, that is, on the side opposite to the adhesion surface 11. The adhesive sheet 2 has flexibility. Therefore, the adhesive sheet 2 can be deformed according to a shape of the biological surface. In addition, the adhesion surface 11 easily follows deformation of the biological surface. As a result, it is possible to prevent the compression device 1 from being unintentionally released from the biological surface.

The adhesive sheet 2 includes a plurality of layers including, for example, a base material layer and an adhesive layer.

The base material layer is formed of, for example, a thin resin sheet. More specifically, the base material layer is formed of, for example, a white spunlace nonwoven fabric of polyester fibers, and has a thickness in a range of 5 µm to 150 µm, and for example, 30 µm. However, a material of the base material layer is not limited to polyester, and, for example, an acrylic polymer, polyethylene, an ethylene-vinyl acetate copolymer, polyurethane, a polyamide derivative, and the like may be used.

The adhesive layer is formed of, for example, an adhesive such as a rubber-based adhesive, an acrylic-based adhesive, and a silicon-based adhesive. The adhesive layer is stacked on the base material layer directly or indirectly with another layer interposed therebetween. The adhesion surface 11 of the adhesive sheet 2 according to the present embodiment is an adhesive layer.

The adhesive sheet 2 may further include another layer in addition to the base material layer and the adhesive layer. The adhesive sheet 2 may include, for example, a surface layer. The surface layer is formed of, for example, a resin having a thickness of about 5 µm to 50 µm. More specifically, examples of a material of the surface layer include polyester, polyamide, polyamideimide, polyethylene, polypropylene, polycarbonate, polyurethane, polyvinyl chloride, and fluororesin. The surface layer is stacked on the base material layer directly or indirectly with another layer interposed therebetween on a side opposite to the adhesive layer with the base material layer interposed therebetween. Therefore, the attachment surface 12 of the adhesive sheet 2 may be a surface layer.

More specifically, the adhesive sheet 2 may be formed of a nonwoven fabric tape having an adhesive agent as an adhesive on one surface thereof. Further, the adhesive sheet 2 may be formed of a double-sided tape in which adhesive layers are provided on both sides of the base material layer. When the adhesive sheet 2 is formed of the double-sided tape, the compression member 3 can be fixed to the adhesive sheet 2 by adhering the holding body 5 of the compression member 3 to one adhesive layer of the adhesive sheet.

The adhesive sheet 2 according to the present embodiment has a substantially C-shaped outer shape in the plan view seen in the thickness direction A. As shown in Figs. 2 and 3, only a part of a lower surface side of the compression member 3 is covered with the adhesive sheet 2 according to the present embodiment. Specifically, only an outer edge region of a lower surface of the compression member 3 is covered with the adhesive sheet 2 according to the present embodiment. The adhesive sheet 2 according to the present embodiment is fixed only to the outer edge region of the lower surface of the compression member 3. In other words, a central region of the lower surface of the compression member 3 in which the inflatable portion 8 of the pressing body 4 of the compression member 3 is located is not covered with the adhesive sheet 2 according to the present embodiment. In addition, the entire outer edge region of the lower surface of the compression member 3 is not covered with the adhesive sheet 2 according to the present embodiment, and a part of the outer edge region is covered with the adhesive sheet 2. That is, a part of the outer edge region of the lower surface of the compression member 3 is not covered with the adhesive sheet 2 according to the present embodiment. According to the present embodiment, a portion of the outer edge region of the lower surface of the compression member 3 which is not covered with the adhesive sheet 2 is a portion adjacent to the receiving portion 6 in the compression member 3.

The adhesion surface 11 of the adhesive sheet 2 is covered with a release sheet in an unused state before being adhered to the biological surface. The release sheet is removed by being released from the adhesion surface 11 by a user when the adhesive sheet 2 is adhered to the biological surface. When the release sheet is removed from the adhesion surface 11 and the adhesion surface 11 is exposed, the adhesion surface 11 of the adhesive sheet 2 is brought into a state of being capable of being adhered to the biological surface (hereinafter, referred to as a "use state" for convenience of description). The release sheet may be formed of, for example, a release paper or a sheet material made of resin. The compression device 1 shown in Figs. 1 to 6 is in the use state in which the release sheet is removed.

### <Pressing Body 4 of Compression Member 3>

As described above, the pressing body 4 of the compression member 3 according to the present embodiment includes the inflatable portion 8 and the extending portion 9. The inflatable portion 8 according to the present embodiment can be inflated by being supplied with a fluid. Specifically, the inflatable portion 8 according to the present embodiment defines accommodation spaces 8a in which a fluid such as gas can be accommodated. The inflatable portion 8 according to the present embodiment includes two balloon portions 8b and 8c that are connected to each other such that the inside of the balloon portion 8b communicates with the inside of the balloon portion 8c. The accommodation spaces 8a according to the present embodiment are internal spaces in which the two balloon portions 8b and 8c communicate with each other. The inflatable portion 8 according to the present embodiment can be inflated by supplying the fluid to the accommodation spaces 8a. The inflatable portion 8 according to the present embodiment includes a plurality of balloon portions, and a configuration of the inflatable portion 8 is not limited to the configuration of the present embodiment. The inflatable portion 8 may include, for example, only one balloon portion.

More specifically, the inflatable portion 8 according to the present embodiment can be inflated toward the downward direction A1 in the thickness direction A by supplying the fluid to the accommodation spaces 8a. The inflatable portion 8 according to the present embodiment is inflated toward the downward direction A1 by changing from the deflated form (see Fig. 5) to the inflated form (see Fig. 6), and is in a posture capable of compressing the biological surface. More specifically, when the fluid is supplied to the accommodation spaces 8a, the inflatable portion 8 receives the reaction force from the lower surface of the holding body 5 and is inflated toward the downward direction A1. The fluid supplied to the accommodation spaces 8a of the inflatable portion 8 is not limited to gas, and may be a liquid.

As shown in Fig. 5, the inflatable portion 8 in the deflated form is disposed along the lower surface of the holding body 5 in a state in which the accommodation spaces 8a are deflated. The accommodation spaces 8a of the inflatable portion 8 communicate with a tube 28 extending to the outside of the holding body 5. A fluid such as air is supplied through the tube 28 to the accommodation spaces 8a of the inflatable portion 8 from a fluid supply device connected to an inflation port as a connection portion 29 provided at an end portion of the tube 28. Accordingly, the inflated state of the inflatable portion 8 can be changed, and the inflatable portion 8 can be changed in form from the deflated form (see Fig. 5) to the inflated form (see Fig. 6).

The inflatable portion 8 according to the present embodiment includes a portion having translucency in the thickness direction A.
"A portion having translucency in the thickness direction A" means a semi-transparent or transparent portion in the thickness direction A. More specifically, the inflatable portion 8 according to the present embodiment includes only the portion having translucency in the thickness direction A. Accordingly, it is preferable that the inflatable portion 8 includes at least the portion having translucency in the thickness direction A. Accordingly, a lower side of the inflatable portion 8 can be seen from an upper side of the inflatable portion 8 through the inflatable portion 8. Therefore, a position of the wound or the like on the biological surface can be visually identified through the inflatable portion 8.

Constituent materials of the inflatable portion 8 of the pressing body 4 include, for example, soft polyvinyl chloride, polyurethane, polyethylene, polypropylene, polyester, ethylene-vinyl acetate copolymer (EVA), silicone, or a material having flexibility obtained by mixing any of these materials.

As described above, the extending portion 9 is extended from the inflatable portion 8 and is wound around the holding body 5. Specifically, the holding body 5 according to the present embodiment defines a through hole 5a penetrating in the thickness direction A. The extending portion 9 according to the present embodiment is wound around the holding body 5 through the through hole 5a. More specifically, the extending portion 9 according to the present embodiment extends from a lower side at which the inflatable portion 8 is located with the holding body 5 interposed therebetween toward an upper side opposite to the lower side. The extending portion 9 according to the present embodiment is wound around the holding body 5 along an inner surface of the holding body 5 that defines the through hole 5a and the upper surface of the holding body 5. In addition, the extending portion 9 is formed with a locking hole 9b on the upper surface side of the holding body 5. A locking protrusion 5b protruding from the upper surface of the holding body 5 is fitted into the locking hole 9b. The extending portion 9 is positioned on the holding body 5 by fitting the locking protrusion 5b into the locking hole 9b. In addition, the extending portion 9 is wound around the holding body 5 from a lower surface side to the upper surface side of the holding body 5 on a receiving portion 6 side with respect to the inflatable portion 8. That is, the through hole 5a according to the present embodiment is located on the receiving portion 6 side with respect to the inflatable portion 8. Therefore, in a cross-sectional view shown in Fig. 5, the inflatable portion 8 and the extending portion 9 that constitute the pressing body 4 according to the present embodiment are curved in a substantially U shape by being wound around the holding body 5. Accordingly, the inflatable portion 8 can be inflated while pivoting about a portion of the extending portion 9 which is connected to the inflatable portion 8 as a hinge portion 9a.

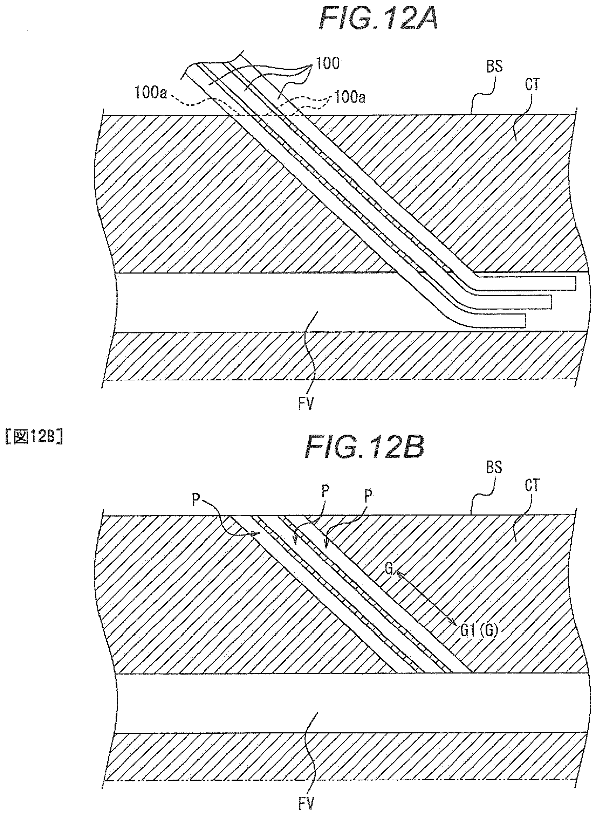

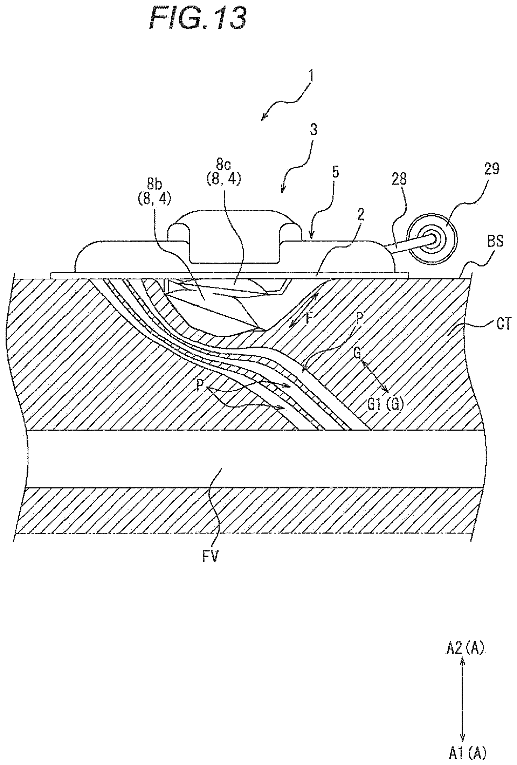

As shown in Fig. 6, the inflatable portion 8 can be inflated not only toward the thickness direction A but also toward a direction inclined with respect to the thickness direction A by pivoting about the hinge portion 9a of the extending portion 9. As described above, the pressing body 4 according to the present embodiment is fixed to the holding body 5 in a state in which the extending portion 9 having a sheet shape is wound around the upper and lower surfaces of the holding body 5 through the through hole 5a of the holding body 5. Therefore, at the time of inflating, the inflatable portion 8 is inflated while pivoting about the hinge portion 9a, which is the portion of the extending portion 9 which is connected to the inflatable portion 8 on a lower side of the through hole 5a, as a pivot center. More specifically, the two balloon portions constituting the inflatable portion 8 according to the present embodiment overlap with each other in the thickness direction A. In addition, one end of each of the two balloon portions is attached to the extending portion 9. That is, one end side of each of the two balloon portions is restrained by the extending portion 9. Therefore, even when the two balloon portions are inflated, the two balloon portions are not separated from each other on the one end side. On the other hand, the other end side of each of the two balloon portions is not restrained at all. Therefore, when the two balloon portions are inflated, the two balloon portions are separated from each other on the other end side. That is, in the two balloon portions constituting the inflatable portion 8 according to the present embodiment, the other end side, which is not attached to the extending portion 9, pivots about a pivot center with the one end side attached to the extending portion 9 as the pivot center. Accordingly, the inflatable portion 8 according to the present embodiment is inflated toward the direction inclined with respect to the thickness direction A. Perforations P (see Fig. 12B) to be described later are easily narrowed or obstructed by inflating the inflatable portion 8 toward the direction inclined with respect to the thickness direction A. The details will be described later (see Fig. 13).

Constituent materials from which the extending portion 9 of the pressing body 4 may be fabricated include, for example, soft polyvinyl chloride, polyurethane, polyethylene, polypropylene, polyester, ethylene-vinyl acetate copolymer (EVA), silicone, or a material having flexibility obtained by mixing any of these materials.

### <Holding Body 5 of Compression Member 3>

The holding body 5 includes a main body portion 31 that is flat and has a substantially quadrangular shape in the plan view, and a pair of gripping plate portions 32 that protrude from the main body portion 31 in the upward direction A2 and that face each other.

The main body portion 31 is formed with the through hole 5a. In addition, the main body portion 31 includes the locking protrusion 5b fitted into the locking hole 9b of the extending portion 9 of the pressing body 4. The inflatable portion 8 of the pressing body 4 is disposed at a lower surface side of a central portion of the main body portion 31 .

The main body portion 31 is fixed to the attachment surface 12 of the adhesive sheet 2. Specifically, only an outer edge portion of a lower surface of the main body portion 31 is fixed to the attachment surface 12 of the adhesive sheet 2 having a substantially C shape in the plan view. The central portion of the lower surface of the main body portion 31 is not covered with the adhesive sheet 2. Therefore, the central portion of the lower surface of the main body portion 31 is not fixed to the attachment surface 12 of the adhesive sheet 2.

The main body portion 31 of the holding body 5 is provided with the marker portion 42. By providing the marker portion 42, the inflatable portion 8 can appropriately compress a compression position on the biological surface to be pressed and compressed by the inflatable portion 8 of the pressing body 4. Specifically, the marker portion 42 according to the present embodiment indicates a place where the biological surface can be compressed with a compression force of a predetermined value or more by the inflatable portion 8 in the top view (see Fig. 2) of the compression device 1 in a state of being attached to the biological surface. Therefore, the compression device 1 is attached to the biological surface such that the marker portion 42 overlaps with a predetermined compression position on the biological surface. Accordingly, the predetermined compression position on the biological surface can be appropriately compressed by the compression device 1. A part of the marker portion 42 according to the present embodiment is provided at a position overlapping with the inflatable portion 8 of the pressing body 4 in the plan view seen in the thickness direction A. Therefore, a part of the marker portion 42 overlapping with the inflatable portion 8 in the thickness direction A may be aligned to overlap with the predetermined compression position on the biological surface in the plan view. Accordingly, by providing the marker portion 42, the compression device 1 can be easily attached to the appropriate position on the biological surface. The marker portion 42 may not be provided at the position overlapping with the inflatable portion 8 in the plan view seen in the thickness direction A, but it is preferable that a part of the marker portion 42 is provided at the position overlapping with the inflatable portion 8 in the plan view seen in the thickness direction A as in the marker portion 42 according to the present embodiment. In addition, the entire marker portion 42 may be provided at the position overlapping with the inflatable portion 8 in the plan view seen in the thickness direction A.

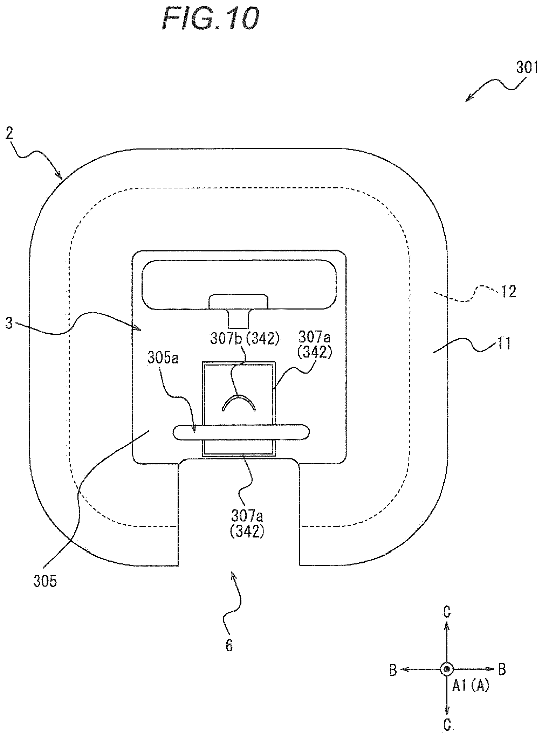

The marker portion 42 according to the present embodiment is provided on an upper surface of the main body portion 31 of the holding body 5. Therefore, it is easy for the health care worker who is operating the compression device 1 to visually identify the marker portion 42 according to the present embodiment. Alternatively, the marker portion 42 may be provided inside the holding body 5. Further, the marker portion 42 may be provided on the lower surface of the main body portion 31 of the holding body 5. The details of the configuration in which the marker portion 42 is provided on the lower surface of the holding body 5 will be described later (see Fig. 10).

The marker portion 42 according to the present embodiment includes the region marker 7a and the position marker 7b. The region marker 7a is a marker that defines a predetermined region in the plan view seen in the thickness direction A. As described above, the region marker 7a according to the present embodiment defines the region in which the inflatable portion 8 can compress the biological surface with the compression force of the first predetermined value or more in the state in which the compression device 1 is attached to the biological surface. In addition, the position marker 7b is a marker indicating a predetermined reference position in the predetermined region defined by the region marker 7a in the plan view seen in the thickness direction A. The position marker 7b according to the present embodiment indicates a reference position for aligning a part of an outer surface of an insertion portion 100a (see Fig. 8A) of the medical insertion member 100 (see Fig. 8A), which is located on the same surface as the biological surface. More specifically, the position marker 7b according to the present embodiment indicates a reference position for aligning the medical insertion member 100 (see Fig. 8A) requiring a particularly large compression force in the predetermined region that can be identified by the region marker 7a.

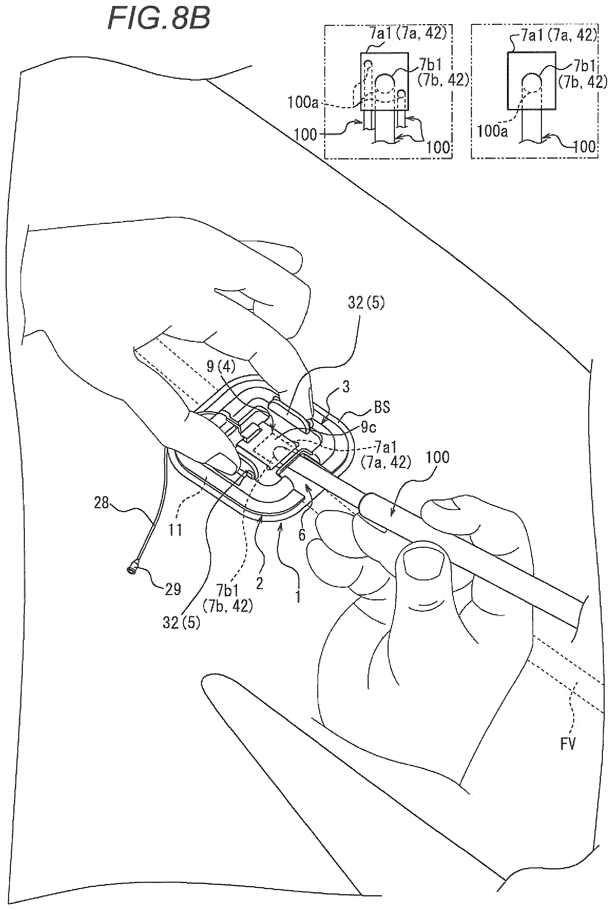

The details will be described later, and as shown in an enlarged manner in a frame of a two-dot chain line on a left side in Fig. 8B, even when a plurality of (three in the frame of the two-dot chain line on the left side in Fig. 8B) the medical insertion members 100 are inserted into the living body, the compression device 1 can be aligned so that all the insertion portions 100a fit within a region of the region marker 7a. That is, by providing the region marker 7a, even when the plurality of medical insertion members 100 are inserted into the living body, the compression device 1 can be easily attached to the appropriate position on the biological surface at which all the insertion portions 100a of the plurality of medical insertion members 100 can be compressed with a predetermined compression force.

The region marker 7a according to the present embodiment is a frame line marker 7a1 surrounding a predetermined region. The frame line marker 7a1 as the region marker 7a can be implemented, for example, by a part of the upper surface of the main body portion 31 of the holding body 5 formed of a resin kneaded with a dye, a colored paint, and the like. A color of the frame line marker 7a1 as the region marker 7a is not particularly limited, and is preferably a green-based color. By using the green-based color, the health care worker or the like can easily and visually identify the frame line marker 7a1 on blood or the biological surface. Therefore, it is easier to align the frame line marker 7a1 as the region marker 7a at the compression position on the biological surface. In addition, the frame line marker 7a1 as the region marker 7a may be constituted by a convex portion protruding from the upper surface of the holding body 5 or a concave portion such as a groove portion formed on the upper surface of the holding body 5.

The frame line marker 7a1 as the region marker 7a according to the present embodiment has a rectangular shape, but the shape is not particularly limited. The frame line marker 7a1 may have, for example, a circular shape, an oval shape, and a polygonal shape other than a square shape. In addition, a part of the frame line marker 7a1 as the region marker 7a according to the present embodiment is cut off by the through hole 5a of the holding body 5, but the configuration is not limited thereto. The frame line marker 7a1 may be provided only on a side opposite to the receiving portion 6 side with respect to the through hole 5a.

The region marker 7a is not limited to the frame line marker 7a1 that defines a predetermined region by a frame line. The region marker 7a may have a configuration in which a predetermined region is bordered by an outer edge of a region filled with a predetermined color. However, by setting the region marker 7a as the frame line marker 7a1, a contour of the predetermined region becomes clear, and thus the health care worker can more easily identify the predetermined region.

As described above, the position marker 7b is located in the predetermined region defined by the region marker 7a in the plan view. As described above, the position marker 7b according to the present embodiment indicates the reference position for aligning a part of the outer surface of the insertion portion 100a (see Fig. 8A). Specifically, the position marker 7b according to the present embodiment indicates the reference position for aligning the medical insertion member 100 (see Fig. 8A) requiring a particularly large compression force in the predetermined region that can be identified by the region marker 7a. That is, the position marker 7b according to the present embodiment indicates the position at which the inflatable portion 8 can compress the biological surface with the compression force of the second predetermined value, which is larger than the first predetermined value, or more in the state in which the compression device 1 is attached to the biological surface. The details will be described later, and the medical insertion member 100 (see Fig. 8A or the like) requiring a large compression force, such as a thick sheath having an outer diameter equal to or higher than a predetermined value, is aligned such that a part of the insertion portion 100a (see Fig. 8A or the like), which is located on the same surface as the biological surface, comes into contact with or overlaps with the position marker 7b in the plan view. The compression device 1 is attached to the biological surface in such an aligned state.

By providing the position marker 7b in addition to the region marker 7a, it is possible to implement a desired compression state by aligning the medical insertion member 100 (see Fig. 8A or the like) requiring a large compression force, such as a large-diameter sheath, with the position marker 7b in the region marker 7a. That is, by providing the position marker 7b in addition to the region marker 7a, the compression device 1 can be easily attached to a more appropriate position on a biological surface BS in consideration of a difference in the compression force within the region of the region marker 7a. That is, the marker portion 42 may include only one of the region marker 7a and the position marker 7b, and preferably includes both of the region marker 7a and the position marker 7b.

The position marker 7b according to the present embodiment is a line segment marker 7b1 extending in the predetermined region defined by the region marker 7a in the plan view seen in the thickness direction A. By using the line segment marker 7b1, regardless of an outer diameter of the medical insertion member 100 (see Fig. 8A or the like) in a state of being inserted into the living body from the biological surface, a part (for example, a part of the outer surface) of the insertion portion 100a (see Fig. 8A or the like) of the medical insertion member 100, which is located on the same surface as the biological surface, can be aligned to come into contact with or overlap with the line segment marker 7b1 in the plan view. Unlike the region marker 7a, the line segment marker 7b1 does not define a predetermined closed region in the plan view. Therefore, even the medical insertion member 100 including the insertion portion 100a that does not match an area or an outer edge contour of the predetermined region defined by the region marker 7a can be aligned with the line segment marker 7b1 by using a part of the insertion portion 100a. Here, the "medical insertion member 100 including the insertion portion 100a that does not match an area or an outer edge contour of the predetermined region defined by the region marker 7a" includes, for example, the medical insertion member 100 having an outer diameter that does not fit in the region defined by the region marker 7a, and the medical insertion member 100 having an outer diameter that can fit a plurality of the medical insertion members 100 in the above described region. Therefore, by providing the line segment marker 7b1, the compression device 1 can be easily attached to the appropriate position on the biological surface regardless of the outer diameter of the medical insertion member 100 inserted into the living body.

The line segment marker 7b1 as the position marker 7b can be implemented, for example, by a part of the upper surface of the main body portion 31 of the holding body 5 formed of a resin kneaded with a dye, a colored paint, and the like. A color of the line segment marker 7b1 as the position marker 7b is not particularly limited, and is preferably a green-based color. By using the green-based color, the health care worker or the like can easily and visually identify the line segment marker 7b1 on blood or the biological surface. The line segment marker 7b1 as the position marker 7b may be constituted by a convex portion protruding from the upper surface of the holding body 5 or a concave portion such as a groove portion formed on the upper surface of the holding body 5.

A shape of the line segment marker 7b1 as the position marker 7b in the plan view is not particularly limited. The line segment marker 7b1 as the position marker 7b according to the present embodiment extends in an arc shape in the plan view. With the line segment marker 7b1 having such a shape, it is easy to align the insertion portion 100a extending in an arc shape in a convex shape in the plan view such that a part of the outer surface of the insertion portion 100a enters a concave portion of the line segment marker 7b1. Therefore, the shape of the line segment marker 7b1 as the position marker 7b in the plan view is not particularly limited, may be an extending linear shape, and the line segment marker 7b1 preferably has a concave portion such as an arc shape or a V shape according to the present embodiment.

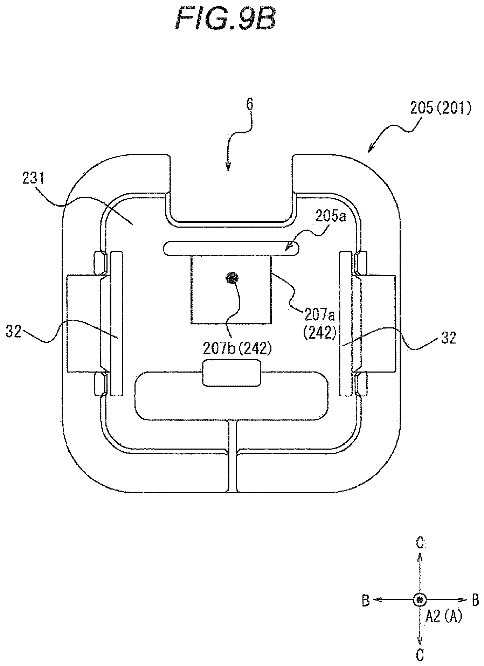

The position marker 7b is not limited to the line segment marker 7b1. The position marker 7b may be, for example, a point marker (see Fig. 9B). Further, the position marker 7b may be formed of an aggregate of point markers. An example of the aggregate of the point markers is a broken line marker formed of point markers arranged in a row.

The marker portion 42 of the main body portion 31 of the holding body 5 may have a non-transparent configuration in the thickness direction A, and is preferably a semi-transparent or transparent portion having translucency in the thickness direction A. Specifically, the region marker 7a and the position marker 7b according to the present embodiment are preferably semi-transparent or transparent portions having translucency in the thickness direction A. Accordingly, the health care worker can visually identify a degree of overlap between the marker portion 42 and the position on the biological surface to be aligned with the marker portion 42.

It is preferable that the periphery of the marker portion 42 of the main body portion 31 of the holding body 5 has translucency in the thickness direction A. Specifically, it is preferable that the holding body 5 according to the present embodiment includes a portion having translucency in the thickness direction A in an adjacent periphery outside the region marker 7a in the plan view seen in the thickness direction A. Further, it is preferable that the holding body 5 according to the present embodiment has translucency in the thickness direction A in the predetermined region defined by the region marker 7a. Accordingly, when the health care worker performs an operation of moving the compression device 1 along the biological surface such that the marker portion 42 overlaps with a predetermined position on the biological surface, the health care worker can move the compression device 1 while visually identifying the position on the biological surface through the portion having translucency in the periphery of the marker portion 42. That is, since the periphery of the marker portion 42 of the main body portion 31 of the holding body 5 has translucency in the thickness direction A, an operation of attaching the compression device 1 to the appropriate position on the biological surface becomes easier.

As described above, the inflatable portion 8 and the extending portion 9 of the pressing body 4 according to the present embodiment also have translucency in the thickness direction A. In the compression device 1 according to the present embodiment, the extending portion 9 of the pressing body 4, the marker portion 42 of the holding body 5 and the periphery thereof, and the inflatable portion 8 of the pressing body 4 are stacked in the thickness direction A. Hereinafter, this part is referred to as a "stacked portion". It is preferable that the stacked portion according to the present embodiment is formed by stacking the portions having translucency. Accordingly, in the compression device 1 according to the present embodiment, it is possible to visually identify one side from the other side in the thickness direction A through the extending portion 9 of the pressing body 4, the marker portion 42 of the holding body 5 and the periphery thereof, and the inflatable portion 8 of the pressing body 4. As a result, according to the compression device 1 according to the present embodiment, the alignment of the marker portion 42 on the biological surface becomes easier. As described above, when the entire stacked portion is constituted by portions having translucency, it is preferable that the marker portion 42 can be identified with respect to all the other layers of the stacked portion in the plan view. Accordingly, the alignment of the marker portion 42 on the biological surface becomes easier.

The marker portion 42 according to the present embodiment is provided at a position adjacent to the receiving portion 6. More specifically, the marker portion 42 according to the present embodiment is adjacent to the receiving portion 6 on a side on which the inflatable portion 8 of the pressing body 4 is located in the plan view of the compression device 1 seen in the thickness direction A. In addition, according to the present embodiment, in the plan view of the compression device 1 seen in the thickness direction A, a maximum width of the marker portion 42 is smaller than a maximum width of the inflatable portion 8 of the pressing body 4. Further, according to the present embodiment, in the plan view of the compression device 1 seen in the thickness direction A, the maximum width of the marker portion 42 is smaller than a maximum width of the extending portion 9 of the pressing body 4. The maximum width of the marker portion 42 according to the present embodiment means a maximum width of the region marker 7a in a width direction B as shown in Fig. 2. In addition, the maximum width of the inflatable portion 8 according to the present embodiment means a maximum width of the inflatable portion 8 in the width direction B as shown in Fig. 2. Further, the maximum width of the extending portion 9 according to the present embodiment means a maximum width of the extending portion 9 in the width direction B as shown in Fig. 2. The width direction B means a direction orthogonal to an arrangement direction C in which the receiving portion 6 and the inflatable portion 8 of the pressing body 4 are arranged in the plan view of the compression device 1 seen in the thickness direction A. A region of the light-transmitting portion 9c in the extending portion 9 according to the present embodiment in the plan view is a region including the entire rectangular region defined by the region marker 7a of the marker portion 42, and extends to the outside of the region marker 7a.

According to the present embodiment, in the plan view of the compression device 1 seen in the thickness direction A, the maximum width of the marker portion 42 is smaller than a minimum width of the receiving portion 6. The minimum width of the receiving portion 6 means a minimum width of the receiving portion 6 in the width direction B as shown in Fig. 2.

As shown in Fig. 2, in the plan view of the compression device 1 seen in the thickness direction A, a position at which the light-transmitting portion 9c of the extending portion 9 and the marker portion 42 overlap with each other is located closer to the receiving portion 6 side than positions of the locking hole 9b and the locking protrusion 5b. With the arrangement, visibility of the marker portion 42 is unlikely to be hindered by the locking hole 9b and the locking protrusion 5b. That is, the health care worker who operates the compression device 1 can easily and visually identify the marker portion 42 during the operation.

A method for providing the marker portion 42 is not particularly limited, and for example, printing, fusion, adhesion, and integral molding can be used.

At least a part of the marker portion 42 according to the present embodiment is provided at a position between the pair of gripping plate portions 32 in the plan view. Accordingly, the health care worker who operates the compression device 1 can easily and visually identify the marker portion 42 in a state of gripping the pair of gripping plate portions 32 to sandwich the pair of gripping plate portions 32 from two sides. Therefore, an operability of the compression device 1 is improved. More specifically, according to the present embodiment, a part of the region marker 7a and the entire position marker 7b of the marker portion 42 are disposed between the pair of gripping plate portions 32 in the plan view.

The pair of gripping plate portions 32 are gripped by the health care worker. By providing the pair of gripping plate portions 32, the compression device 1 can be easily held. Therefore, it is possible to improve the operability for the health care worker.

Examples of a material of the holding body 5 according to the present embodiment include a resin material. Examples of the resin material include thermoplastic resins used in injection molding such as ABS resin, AS resin, polyethylene, polypropylene, polystyrene, polyvinyl chloride, polyvinylidene chloride resin, polyphenylene oxide, thermoplastic polyurethane, polymethylene methacrylate, polyoxyethylene, fluororesin, polycarbonate, polyamide, acetal resin, acrylic resin, and polyethylene terephthalate, and thermosetting resins such as phenol resin, epoxy resin, silicone resin, and unsaturated polyester.

It is preferable that at least the central portion of the main body portion 31 of the holding body 5 is formed of a material having ultrasonic transmissibility. In addition, it is preferable that the pressing body 4 is also formed of a material having ultrasonic transmissibility. Further, as the fluid supplied to the accommodation spaces 8a of the inflatable portion 8 of the pressing body 4, a fluid having ultrasonic transmissibility such as water or gel is used. Accordingly, an obstructed state of the blood vessel made by the compression device 1 can be detected by an ultrasonic device. The details will be described later.

### <Compression Method Using Compression Device 1>

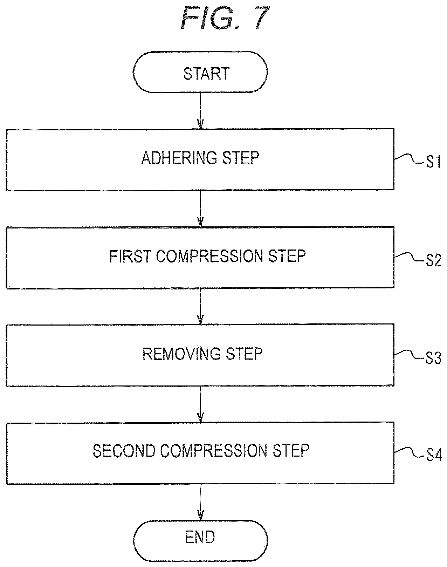

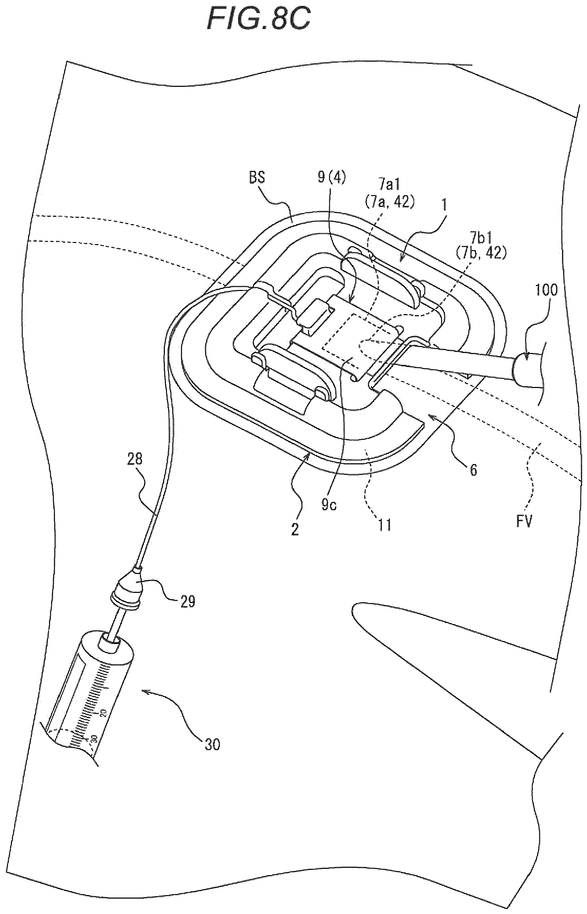

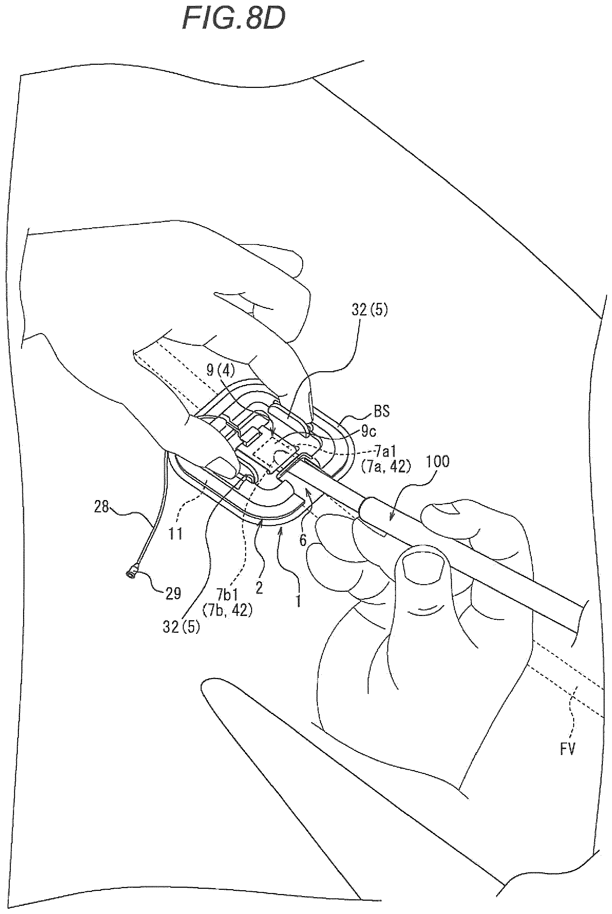

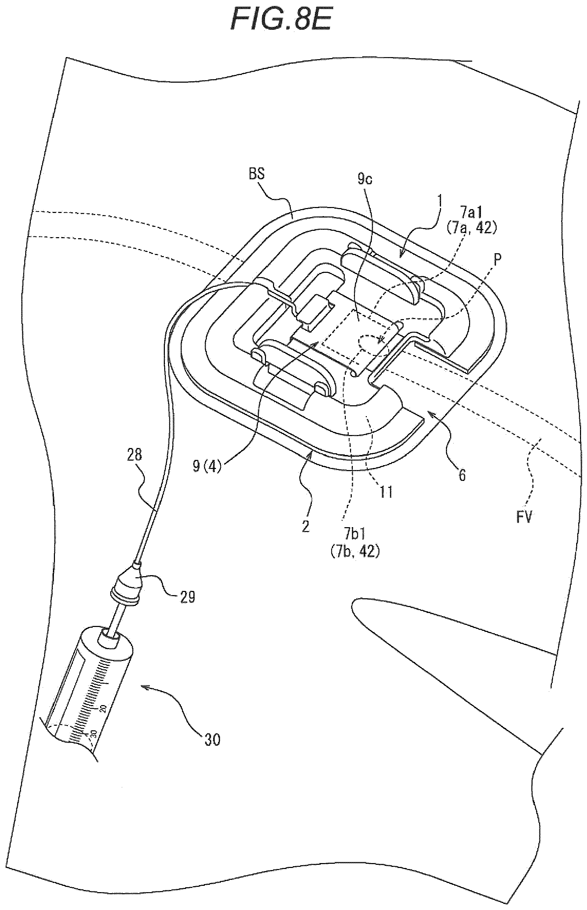

Next, a method for compressing a biological surface using the compression device 1, the method including an example of a method for adhering the compression device 1 according to this disclosure, will be described. Fig. 7 is a flowchart showing an example of the method for compressing the biological surface. The compression method shown in Fig. 7 includes an adhering step S1, a first compression step S2, a removing step S3, and a second compression step S4. Figs. 8A and 8B are diagrams showing an outline of the adhering step S1. Fig. 8C is a diagram showing an outline of the first compression step S2. Fig. 8D is a diagram showing an outline of the removing step S3. Fig. 8E is a diagram showing an outline of the second compression step S4.

The compression method shown in Figs. 7 to 8E is a method for compressing the biological surface BS to narrow or obstruct a perforation leading from the biological surface to a vein without obstructing the vein. The perforation is formed by removing a sheath as the medical insertion member 100 in a state of being inserted into a vein such as a femoral vein from the biological surface BS through a connective tissue. By the compression method shown here, bleeding can be stopped after the sheath as the medical insertion member 100 is removed. First, the perforation formed after the medical insertion member 100 is removed will be described with reference to Figs. 12A and 12B. Fig. 12A shows a state in which the sheath as the medical insertion member 100 is inserted into a femoral vein FV from the biological surface BS through a connective tissue CT. Fig. 12A shows three sheaths as the medical insertion members 100, and the number of sheaths may be two or less, or may be four or more. Fig. 12B shows a state after the sheaths as the medical insertion members 100 are removed from the state shown in Fig. 12A. As shown in Fig. 12B, when the sheaths as the medical insertion members 100 are removed, the perforations P are formed between the biological surface BS and the femoral vein FV. In the compression method shown in Figs. 7 to 8E, the perforations P can be narrowed or obstructed without obstructing the femoral vein FV. Therefore, even when bleeding from a vein located at a deep position from the biological surface is stopped, bleeding can be stopped relatively efficiently without narrowing or obstructing the vein itself. Hereinafter, the details of the steps S1 to S4 will be described with reference to Figs. 8A to 8E.

Fig. 8A shows a state in which the sheath as the medical insertion member 100 is inserted from the biological surface BS into the femoral vein FV (see Figs. 12A and 12B). Fig. 8B shows a state in which the attachment of the compression device 1 to a predetermined position on the biological surface BS is completed in a state in which the sheath as the medical insertion member 100 is inserted into the living body. In addition, Fig. 8B shows the details of the alignment between the marker portion 42 and the insertion portion 100a of the medical insertion member 100 in the plan view in an enlarged manner within frames of two-dot chain lines. Figs. 8A and 8B show the use state of the compression device 1 after the release sheet is released from the adhesion surface 11.

As shown in Figs. 8A and 8B, the compression device 1 is attached to the biological surface by adhering the adhesion surface 11 of the adhesive sheet 2 to the biological surface. Specifically, as shown in Fig. 8B, the adhesive sheet 2 is adhered to the biological surface BS in a state in which a portion of the sheath as the medical insertion member 100 inserted into the living body from the biological surface BS, the portion being exposed to the outside from the biological surface BS, is received by the receiving portion 6. Further, as shown in Fig. 8B, the insertion portion 100a of the sheath as the medical insertion member 100, which is located on the same surface as the biological surface BS, is aligned with the marker portion 42 (see the frames of the two-dot chain lines in Fig. 8B). The adhesive sheet 2 is adhered to the biological surface BS in such an aligned state.

More specifically, as shown in Fig. 8B, according to the present embodiment, entirety of the insertion portion 100a of the sheath as the medical insertion member 100, which is located on the same surface as the biological surface BS, is aligned within the region marker 7a of the marker portion 42. The adhesive sheet 2 is adhered to the biological surface BS in such an aligned state. By providing the region marker 7a, as shown in the enlarged manner in the frame of the two-dot chain line on a left side in Fig. 8B, even when a plurality of (three in the frame of the two-dot chain line on the left side in Fig. 8B) the medical insertion members 100 are inserted into the living body, all the insertion portions 100a can be aligned to fit within the region of the region marker 7a. Accordingly, the compression device 1 can be easily attached to an appropriate position on the biological surface BS at which all the insertion portions 100a of the plurality of medical insertion members 100 can be compressed with a predetermined compression force.

As shown in Fig. 8B, according to the present embodiment, a thin sheath having an outer diameter less than a predetermined value is not aligned with the position marker 7b in the region marker 7a (see the frame of the two-dot chain line on the left side in Fig. 8B), and a part of the outer surface of the insertion portion 100a of a thick sheath having an outer diameter equal to or higher than the predetermined value is aligned with the position marker 7b in the region marker 7a (see the frames of the two-dot chain lines on the left and right sides in Fig. 8B). As described above, the position marker 7b according to the present embodiment indicates a position in the region of the region marker 7a at which a particularly large compression force can be obtained. Therefore, for example, even in a case of a large-diameter sheath or the like that requires a large compression force, a predetermined compression force can be secured. In addition, for example, even when both the large-diameter sheath that requires the large compression force and a small-diameter sheath thinner than the large-diameter sheath are provided (see the frame of the two-dot chain line on the left side in Fig. 8B), the large-diameter sheath is aligned with the position marker 7b in the region marker 7a, and the small-diameter sheath is not aligned with the position marker 7b in the region marker 7a, and thus a desired compression force required for each sheath can be implemented based on a difference in compression forces in the region marker 7a. Accordingly, after various alignments are performed, the adhesive sheet 2 is adhered to the biological surface BS. By providing the position marker 7b in addition to the region marker 7a, the compression device 1 can be easily attached to a more appropriate position on the biological surface BS in consideration of the difference in the compression forces within the region of the region marker 7a.

As described above, the position marker 7b according to the present embodiment is the line segment marker 7b1 that is curved and extends in the arc shape in the plan view seen in the thickness direction A. Therefore, according to the present embodiment, a part of the outer surface of the insertion portion 100a of the medical insertion member 100 is aligned to come into contact with or overlap with the line segment marker 7b1 as the position marker 7b in the plan view. The compression device 1 is attached to the biological surface BS in such an aligned state.

Next, as shown in Fig. 8C, a syringe 30 as the fluid supply device is connected to the connection portion 29 of the tube 28. Through the tube 28, air is supplied to the accommodation spaces 8a (see Fig. 6) of the inflatable portion 8 of the pressing body 4 of the compression device 1 to inflate the inflatable portion 8. Accordingly, before the sheath as the medical insertion member 100 is removed from the biological surface BS, the vicinity of a wound on the biological surface BS can be compressed in advance. In other words, compression on the biological surface BS is started in a state in which the sheath as the medical insertion member 100 is inserted into the femoral vein FV as a vein from the biological surface BS through the connective tissue CT (see Figs. 12A and 12B). In this way, the medical insertion member 100 is compressed before being removed from the biological surface BS. Accordingly, immediately after the sheath as the medical insertion member 100 is removed, the biological surface BS can be compressed. Therefore, the perforations P (see Fig. 12B) extending from the biological surface BS to the femoral vein FV (see Figs. 12A and 12B) can be narrowed or obstructed immediately after the sheaths are removed.

Next, as shown in Fig. 8D, the sheath as the medical insertion member 100 is removed from the biological surface BS. The perforations P shown in Fig. 12B are formed by removing the sheaths. When the biological surface BS is not compressed at all in this state, bleeding from the femoral vein FV to the outside of the living body through the perforations P and the wound on the biological surface BS occurs. However, in the compression method shown here, as shown in Fig. 8C, the biological surface BS is compressed in advance before the sheath as the medical insertion member 100 is removed from the biological surface BS. Therefore, immediately after the sheaths are removed, the biological surface BS can be compressed to narrow or obstruct the perforations P (see Fig. 12B), and the amount of bleeding immediately after the sheaths are removed can be reduced.

Next, as shown in Fig. 8E, the syringe 30 as the fluid supply device is connected again to the connection portion 29 of the tube 28. Air is supplied again to pressurize the accommodating spaces 8a of the inflatable portion 8 of the compression device 1 through the tube 28, or the air is evacuated from the accommodating spaces 8a to reduce the pressure. In other words, a compression force on the biological surface BS is adjusted after the sheath as the medical insertion member 100 is removed. Accordingly, by adjusting the compression force on the biological surface BS and further narrowing or obstructing the perforations P (see Fig. 12B) without obstructing the femoral vein FV (see Figs. 12A and 12B), the amount of bleeding can be greatly reduced or the bleeding can be stopped.

More specifically, when the bleeding is confirmed after the sheaths are removed, the compression force is slowly increased to increase the pressure until the bleeding is stopped. On the other hand, when the bleeding is confirmed to be stopped after the sheaths are removed, the compression force is slowly reduced to reduce the pressure until the bleeding is confirmed. After the bleeding is confirmed, the compression force is slowly increased to increase the pressure until the bleeding is stopped. Accordingly, it is possible to prevent the obstruction of the femoral vein FV (see Figs. 12A and 12B) due to excessive pressurization.

Whether the biological surface BS is appropriately compressed may be detected using the ultrasonic device. Specifically, the pressing body 4 and the holding body 5 are formed of a material having ultrasonic transmissibility, and by supplying the fluid having ultrasonic transmissibility such as water to the inflatable portion 8 of the pressing body 4, a compression state made by the compression device 1 can be diagnosed by ultrasounds. That is, the ultrasonic device can detect whether the femoral vein FV (see Figs. 12A and 12B) is obstructed. The compression force of the compression device 1 may be adjusted based on a diagnosis result by the ultrasonic device.

By maintaining the compression state for several hours (for example, 2 to 6 hours) as it is, the bleeding can be stopped. After the bleeding is stopped, the compression device 1 is detached from the biological surface BS by releasing the adhesion surface 11 of the adhesive sheet 2 from the biological surface BS.

In the compression method shown here, the perforations P (see Fig. 12B) are narrowed or obstructed without obstructing the femoral vein FV (see Figs. 12A and 12B). When stopping the bleeding from the vein, the bleeding can be stopped by narrowing or obstructing the perforations P (see Fig. 12B). On the other hand, for example, when stopping bleeding from a femoral artery, even when only the perforations are obstructed, the blood leaks and spreads in the connective tissue CT (see Figs. 12A and 12B), and thus the bleeding cannot be stopped. When stopping the bleeding from the femoral artery, it is necessary to take a large measure, such as a method for strongly compressing the artery itself until the artery is narrowed or obstructed, or a method for obstructing a hole of an artery wall.

Therefore, in the compression method, it is preferable to compress the biological surface BS to a position at which a compression depth from the biological surface BS is 5 mm to 20 mm. By setting the compression depth within the above-mentioned range, it is easy to implement the compression state in which the perforations P (see Fig. 12B) are narrowed or obstructed without obstructing the vein. The compression depth is more preferably 5 mm to 15 mm, and even more preferably 8 mm to 12 mm.

In the compression method, it is preferable to compress the biological surface BS at 10 g/cm² to 600 g/cm² from the biological surface BS. Compression pressure is pressure after the sheath as the medical insertion member 100 is removed, and does not mean the above-mentioned compression force before the sheath is removed. By setting the compression pressure in the above-mentioned range, it is easy to implement the compression state in which the perforations P (see Fig. 12B) are narrowed or obstructed without obstructing the vein. The compression pressure is more preferably 50 g/cm² to 400g/cm², and even more preferably 100 g/cm² to 300 g/cm².

It is preferable to compress the biological surface BS along a direction orthogonal to an extending direction of the perforations P (see Fig. 12B). The phrase "compressing the biological surface BS along a direction orthogonal to an extending direction of the perforations" means not only compressing only in the direction orthogonal to the extending direction of the perforations but also compressing in a direction inclined at an angle equal to or less than a predetermined angle (for example, 30 degrees or less) with respect to the direction orthogonal to the extending direction of the perforations. The compression device 1 according to the present embodiment can compress the biological surface BS along the direction orthogonal to the extending direction of the perforations P (see Fig. 12B).

Specifically, as described above, the inflatable portion 8 of the pressing body 4 according to the present embodiment can be inflated toward the direction inclined with respect to the thickness direction A. Accordingly, the biological surface can be compressed along the direction orthogonal to the extending direction of the perforations P (see Fig. 12B). Specifically, as shown in Figs. 12A and 12B, the sheath as the medical insertion member 100 is inserted not in a direction orthogonal to the biological surface BS (the same direction as the thickness direction A) but in a direction inclined to one side with respect to the direction orthogonal to the biological surface BS. Therefore, as shown in Fig. 12B, the extending direction of the perforations P is also inclined with respect to the direction orthogonal to the biological surface BS. Therefore, when the inflatable portion 8 can be inflated in a direction inclined to a side opposite to the extending direction of the perforations P (hereinafter, may be referred to as an "inclination direction F") with respect to the thickness direction A which is the direction orthogonal to the biological surface BS, the biological surface BS is easily compressed along the direction orthogonal to the extending direction of the perforations P. Accordingly, it is easy to implement the compression device 1 that narrows or obstructs the perforations P without obstructing the vein such as the femoral vein FV in Figs. 12A and 12B. Fig. 13 is a diagram showing the state in which the perforations P shown in Fig. 12B are narrowed or obstructed by the compression device 1. As shown in Fig. 13, the perforations P are more easily narrowed or obstructed without further obstructing the vein such as the femoral vein FV by the compression device 1.

According to the compressing method shown in Figs. 7 to 8E, the bleeding can be stopped by narrowing or obstructing the perforations P (see Fig. 12B) without obstructing the vein such as the femoral vein FV. In particular, by implementing the compression method using the compression device 1, it is possible to stop the bleeding by a simple method without compression by a hand of the health care worker or using a large-scale hemostasis device.

### <Compression of Compression Device 1 on Biological Surface >

As shown in Fig. 13, in the compression device 1, the inflatable portion 8 of the pressing body 4 of the compression member 3 can compress the biological surface toward the inclination direction F inclined with respect to a vertical direction (in Fig. 13, the vertical direction is the same direction as the thickness direction A, and is an upward-downward direction in Fig. 13. Hereinafter, it is simply referred to as a "vertical direction".) perpendicular to the biological surface BS in a state in which the adhesive sheet 2 is attached to the living body. Accordingly, as shown in Fig. 13, the perforations P are easily narrowed or obstructed without obstructing the vein such as the femoral vein FV.

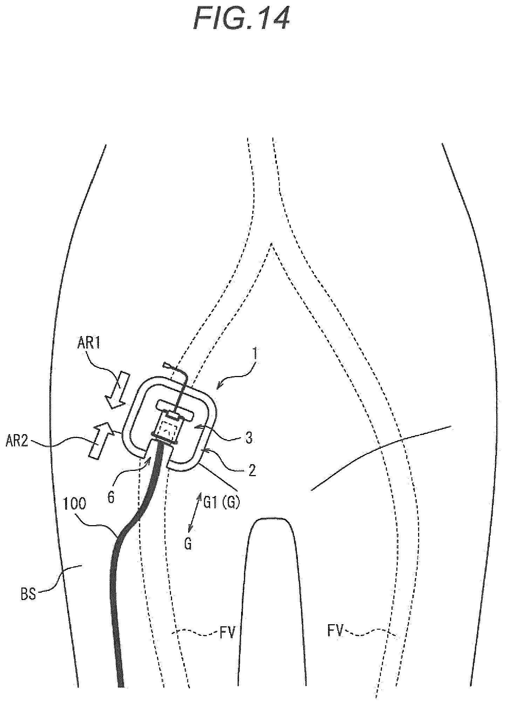

Fig. 14 is a front view of the state shown in Fig. 13 as seen from a biological surface BS side. In other words, Fig. 14 shows a front view of the biological surface BS at a position compressed by the compression device 1. Here, the phrase "front view of the biological surface at a position compressed by the compression device" means a state in which a portion of the biological surface to be compressed by the compression device is seen from a direction perpendicular to the portion before the compressing. Fig. 14 shows a front view of an inguinal region. In the front view shown in Fig. 14, a direction in which the biological surface BS is compressed (see a white arrow "AR1" in Fig. 14) is opposite to an insertion direction G1 (see a white arrow "AR2" in Fig. 14) of the sheath from the biological surface BS toward the vein in an extending direction G of the perforations P. That is, the direction in which the compression device 1 compresses the biological surface BS is opposite to the insertion direction G1 of the sheath in the front view shown in Fig. 14. Accordingly, the perforations P (see Figs. 12B and 13) are easily narrowed or obstructed without obstructing the vein such as the femoral vein FV.

In other words, as shown in Fig. 13, the extending direction G of the perforations P is inclined with respect to the biological surface BS and is also inclined with respect to the vertical direction (upward-downward direction in Fig. 13) perpendicular to the biological surface BS. In addition, as shown in Fig. 13, a compression direction of the compression device 1 on the biological surface BS is also inclined with respect to the biological surface BS and also inclined with respect to the vertical direction (upward-downward direction in Fig. 13) perpendicular to the biological surface BS. Further, as shown in Fig. 13, the extending direction G of the perforations P is inclined to a side opposite to the inclination direction F as the compression direction of the compression device 1 on the biological surface with respect to the vertical direction (upward-downward direction in Fig. 13). That is, the compression of the compression device 1 on the biological surface is executed such that the compression direction intersects with the extending direction G of the perforations P. Accordingly, the perforations P can be efficiently narrowed or obstructed.

### [Second Embodiment]

Next, a compression device 101 according to a second embodiment will be described. Here, a difference from the compression device 1 (see Fig. 1 or the like) will be mainly described, and the description of the common configurations will be omitted.

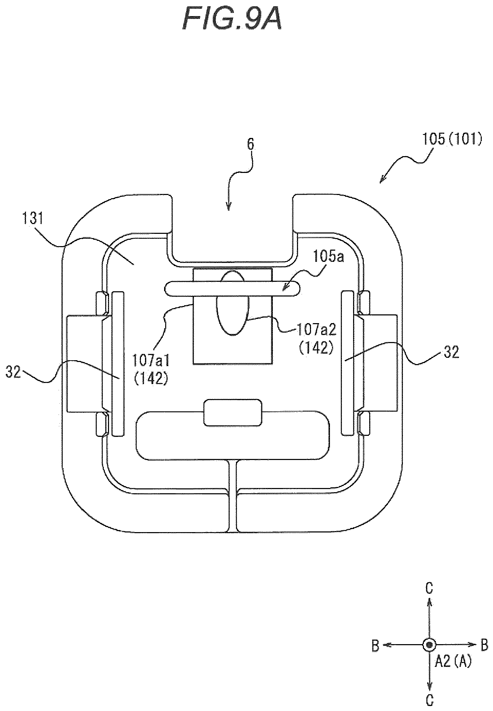

Fig. 9A is a top view of a holding body 105 of the compression device 101 according to the present embodiment. The compression device 101 according to the present embodiment is different from the compression device 1 in a configuration of a marker portion provided in a main body portion of a holding body.

As shown in Fig. 9A, a marker portion 142 is provided in a main body portion 131 of the holding body 105. The marker portion 142 includes two region markers. One region marker is the same frame line marker as the region marker 7a according to the first embodiment. The other region marker is a frame line marker that further defines a part of a region defined by the one region marker. Hereinafter, for convenience of description, the one region marker that is the same as the region marker 7a according to the first embodiment is referred to as a "first region marker 107a1". In addition, the other region marker is referred to as a "second region marker 107a2".

Since the first region marker 107a1 is the same as the region marker 7a according to the first embodiment, the description thereof is omitted here.

Within a predetermined region defined by the first region marker 107a1 in a plan view seen in the thickness direction A, the second region marker 107a2 further defines a part of the predetermined region. The first region marker 107a1 means, for example, a region in which a compression force of a first predetermined value or more can be implemented. The second region marker 107a2 means, for example, a region in which a compression force of a second predetermined value, which is larger than the first predetermined value, or more can be implemented. Accordingly, by providing the first region marker 107a1 and the second region marker 107a2, it is possible to switch region markers for aligning the insertion portion 100a (see Fig. 8A or the like) of the medical insertion member 100 (see Fig. 8A or the like) according to the outer diameter and the number of the medical insertion member 100 (see Fig. 8A or the like) and a necessary compression force. When the second region marker 107a2 is a region in which a large compression force as described above can be obtained, the second region marker 107a2 may be, for example, a priority marker that is aligned in preference to the first region marker 107a1. That is, only when the insertion portion 100a (see Fig. 8A or the like) of the medical insertion member 100 (see Fig. 8A or the like) does not fit within the predetermined region defined by the second region marker 107a2, a region outside the second region marker 107a2 and inside the first region marker 107a1 in the plan view may be used for alignment. If the insertion portion 100a (see Fig. 8A or the like) of the medical insertion member 100 (see Fig. 8A or the like) can be disposed only in the region of the second region marker 107a2, a fluid amount supplied to the inflatable portion 8 (see Fig. 6 or the like) of the pressing body 4 for stopping bleeding can be reduced. Accordingly, it is possible to prevent the release of the adhesive sheet 2 (see Fig. 6 or the like) from a biological surface.

Both a part of the first region marker 107a1 and a part of the second region marker 107a2 according to the present embodiment are cut off by a through hole 105a of the holding body 105 in the plan view, but this disclosure is not limited to the configuration. The first region marker 107a1 and the second region marker 107a2 may be provided on only one side with respect to the through hole 105a of the holding body 105 in the plan view.

The frame line marker as the first region marker 107a1 according to the present embodiment has a rectangular shape, but the shape is not particularly limited. The frame line marker as the first region marker 107a1 may have, for example, a circular shape, an oval shape, and a polygonal shape other than a square shape.

The frame line marker as the second region marker 107a2 according to the present embodiment has an oval shape, but the shape is not particularly limited. The frame line marker as the second region marker 107a2 may have, for example, a circular shape and a polygonal shape such as a rectangular shape.

### [Third Embodiment]

Next, a compression device 201 according to a third embodiment will be described. Here, a difference from the compression device 1 (see Fig. 1 or the like) will be mainly described, and the description of the common configurations will be omitted.

Fig. 9B is a top view of a holding body 205 of the compression device 201 according to the present embodiment. The compression device 201 according to the present embodiment is different from the compression device 1 in a configuration of a marker portion provided in a main body portion of a holding body.

As shown in Fig. 9B, a marker portion 242 is provided in a main body portion 231 of the holding body 205. The marker portion 242 includes a region marker 207a and a position marker 207b.

The region marker 207a according to the present embodiment is a concave marker formed by a concave portion formed on an upper surface of the main body portion 231 of the holding body 205. As shown in Fig. 9B, the concave marker as the region marker 207a is bordered in a rectangular shape by an edge portion of the concave portion in a plan view, and defines a predetermined region inside the region marker 207a.

The position marker 207b according to the present embodiment is a point marker disposed in the predetermined region defined by the region marker 207a in the plan view. The point marker as the position marker 207b indicates a predetermined reference position in the plan view. The predetermined reference position can be, for example, a position that overlaps with the inflatable portion 8 (see Fig. 6 or the like) in the plan view.

The concave marker as the region marker 207a according to the present embodiment has the rectangular shape in the plan view, but the shape is not particularly limited. The concave marker as the region marker 207a may have, for example, a circular shape, an oval shape, and a polygonal shape other than a square shape in the plan view.

The point marker as the position marker 207b according to the present embodiment has a circular shape in the plan view, but the shape is not particularly limited. The point marker as the position marker 207b may have, for example, an oval shape and a polygonal shape such as a rectangular shape in the plan view.

The marker portion 242 according to the present embodiment is provided on only one side with respect to a through hole 205a of the holding body 205 in the plan view, but is not limited to the configuration. The marker portion 242 may be provided on both sides with the through hole 205a interposed therebetween in the plan view.

### [Fourth Embodiment]

Next, a compression device 301 according to a fourth embodiment will be described. Here, a difference from the compression device 1 (see Fig. 1 or the like) will be mainly described, and the description of the common configurations will be omitted.

Fig. 10 is a bottom view of the compression device 301 according to the present embodiment in a state in which the pressing body 4 (see Fig. 1 or the like) is removed. The compression device 301 according to the present embodiment is different from the compression device 1 in a configuration of a marker portion provided in a holding body.

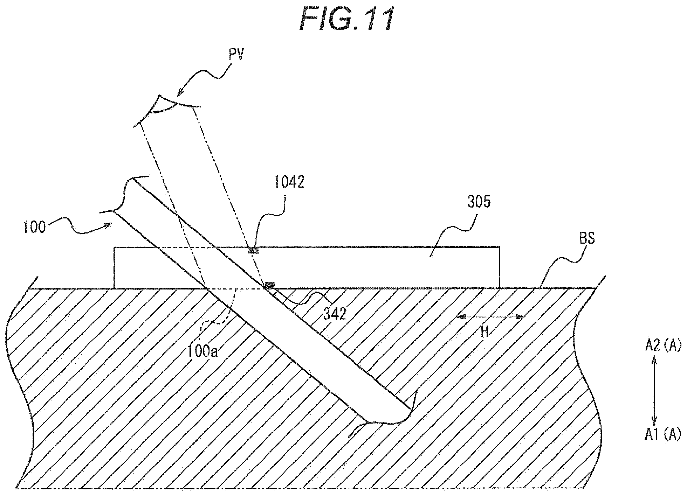

A marker portion 342 according to the present embodiment is provided on a lower surface of a holding body 305. Fig. 11 is a schematic diagram illustrating a state in which a part of the outer surface of the insertion portion 100a of the medical insertion member 100, which is located on the same surface as a biological surface, is positioned with respect to the marker portion 342 provided on the lower surface of the holding body 305. In other words, Fig. 11 is a schematic diagram illustrating an effect of the marker portion 342 provided on the lower surface of the holding body 305. For convenience of description, Fig. 11 shows a marker portion 1042 provided on an upper surface of the holding body 305 for comparison. As shown in Fig. 11, by providing the marker portion 342 on the lower surface of the holding body 305, it is possible to prevent occurrence of positional variation caused by a thickness of the holding body 305 in the thickness direction A, as compared with the configuration in which the marker portion 1042 is provided on the upper surface of the holding body 305. That is, when the marker portion 1042 is provided on the upper surface of the holding body 305, the marker portion 1042 is separated from the biological surface on a lower surface side of the holding body 305 by at least the thickness of the holding body 305. Therefore, when the marker portion 1042 is aligned at a predetermined position on the biological surface at an erroneous viewpoint (for example, a viewpoint PV shown in Fig. 11) deviated from a plan view, the marker portion 1042 and a predetermined position on the biological surface (a part of the outer surface of the insertion portion 100a in Fig. 11) may be positionally deviated in an in-plane direction H of the biological surface. On the other hand, according to the present embodiment, since the marker portion 342 is provided on the lower surface of the holding body 305, the positional deviation due to a difference in viewpoint is unlikely to occur. That is, by providing the marker portion 342 on the lower surface of the holding body 305, the compression device 301 can be more easily attached to an appropriate position on the biological surface.

As shown in Fig. 10, the marker portion 342 includes a region marker 307a and a position marker 307b.

The region marker 307a according to the present embodiment is a frame line marker that defines a predetermined region by a rectangular groove portion formed on the lower surface of the holding body 305. In addition, the position marker 307b according to the present embodiment is a line segment marker formed by a groove portion extending in an arc shape formed on the lower surface of the holding body 305.

A part of the region marker 307a according to the present embodiment is cut off by a through hole 305a of the holding body 305 in the plan view, but the region marker 307a is not limited to the configuration. The region marker 307a may be provided on only one side with respect to the through hole 305a of the holding body 305 in the plan view.

The frame line marker as the region marker 307a according to the present embodiment has a rectangular shape in the plan view, but the shape is not particularly limited. The frame line marker as the region marker 307a may have, for example, a circular shape, an oval shape, and a polygonal shape other than a square shape in the plan view.

The line segment marker as the position marker 307b according to the present embodiment extends in the arc shape in the plan view, but the shape is not particularly limited. The line segment marker as the position marker 307b may extend in, for example, a linear shape or a V shape in the plan view.

The compression device and the method for adhering the compression device according to this disclosure are not limited to the specific configurations and the methods shown in the first embodiment to the fourth embodiment, and various modifications and changes may be made without departing from the description of the claims. In addition, a compression device formed by combining the configurations of the portions of the compression device shown in the first embodiment to the fourth embodiment also belongs to the technical scope of this disclosure.

### Industrial Applicability

This disclosure relates to a compression device and a method for adhering a compression device.

### Reference Signs List

1, 101, 201, 301 compression device
2 adhesive sheet
3 compression member
4 pressing body
5, 105, 205, 305 holding body
5a, 105a, 205a, 305a through hole
5b locking protrusion
6 receiving portion
7a, 207a, 307a region marker
7a1 frame line marker (region marker)
7b, 207b, 307b position marker
7b1 line segment marker (position marker)
8 inflatable portion
8a accommodation space
8b, 8c balloon portion
9 extending portion
9a hinge portion
9b locking hole
9c light-transmitting portion
11 adhesion surface
12 attachment surface
28 tube
29 connection portion
30 syringe
31, 131, 231 main body portion
32 a pair of gripping plate portions
42, 142, 242, 342 marker portion
100 medical insertion member
100a insertion portion
107a1 first region marker
107a2 second region marker
1042 marker portion
A thickness direction
A1 downward direction
A2 upward direction
B width direction
C arrangement direction
F inclination direction
G extending direction of perforation
G1 insertion direction of sheath
H in-plane direction of biological surface
BS biological surface
CT connective tissue
FV femoral vein
P perforation
PV viewpoint
X1 first portion of adhesive sheet
X2 second portion of adhesive sheet

## Claims

1. A compression device comprising:
an adhesive sheet having an adhesion surface configured to be adhered to a biological surface;
a compression member that is fixed to the adhesive sheet and that is configured to compress the biological surface;
the compression member including
a pressing body configured to press the biological surface by extending in a thickness direction of the adhesive sheet, and
a holding body that is fixed to the adhesive sheet on a side opposite to the adhesion surface and that holds the pressing body so that the pressing body is extendable in the thickness direction,
the pressing body includes
an inflatable portion that is inflatable in the thickness direction and configured to be disposed between the biological surface and the holding body when the adhesion surface of the adhesive sheet is adhered to the biological surface, and
a flexible extending portion that extends from the inflatable portion, that is wound around the holding body to extend from the inflatable portion to an upper surface side of the holding body that is on a side of the holding body opposite the inflatable portion with the holding body interposed therebetween, and that is locked to the holding body on the upper surface side of the holding body,
the extending portion including a light-transmitting portion that is translucent in the thickness direction at a position at which an upper surface of the holding body is covered, and
the holding body includes, at a position overlapping with the light-transmitting portion of the extending portion in a plan view seen in the thickness direction, a marker portion configured to be visually identified.

2. The compression device according to Claim 1, wherein
the holding body defines a through hole penetrating in the thickness direction, and
the extending portion is wound around the holding body through the through hole.

3. The compression device according to Claim 1 or 2, wherein the holding body comprising a receiving portion configured to receive a medical tube member, the receiving member being provided outside an outer edge of the holding body as seen in the plan view in the thickness direction, being a region in which the adhesive sheet is not disposed or a region defined by a concave portion in an outer edge of the adhesive sheet,
the extending portion is wound around the holding body on a receiving portion side with respect to the inflatable portion, and
the inflatable portion is inflated while pivoting about a portion connected to the extending portion as a hinge portion.

4. The compression device according to any one of Claims 1 to 3, wherein
the marker portion includes a region marker that defines a predetermined region in the plan view seen in the thickness direction.

5. The compression device according to Claim 4, wherein
the region marker is a frame line marker that surrounds the predetermined region.

6. The compression device according to Claim 4 or 5, wherein
the holding body is translucent in the thickness direction in the predetermined region defined by the region marker.

7. The compression device according to any one of Claims 4 to 6, wherein
the marker portion includes a position marker indicating a predetermined reference position in the predetermined region defined by the region marker in the plan view seen in the thickness direction.

8. The compression device according to Claim 7, wherein
the position marker is an arc-shaped line segment marker extending in the predetermined region defined by the region marker in the plan view seen in the thickness direction.

9. A method for adhering a compression device to a biological surface, wherein
the compression device includes
an adhesive sheet having an adhesion surface configured to be adhered to the biological surface, and
a compression member that is fixed to the adhesive sheet and that is configured to compress the biological surface,
the compression member includes
a pressing body configured to press the biological surface by extending in a thickness direction of the adhesive sheet, and
a holding body that is fixed to the adhesive sheet on a side opposite to the adhesion surface and that is configured to hold the pressing body to be extendable in the thickness direction,
the pressing body includes
an inflatable portion that is disposed between the biological surface and the holding body in a state in which the adhesion surface of the adhesive sheet is adhered to the biological surface and that is inflatable in the thickness direction, and
a flexible extending portion that extends from the inflatable portion, that is wound around the holding body to extend from the inflatable portion to an upper surface side of the holding body on a side opposite to the inflatable portion with the holding body interposed therebetween, and that is locked to the holding body on the upper surface side of the holding body,
the extending portion includes a light-transmitting portion having translucency in the thickness direction at a position at which an upper surface of the holding body is covered,
the holding body includes, at a position overlapping with the light-transmitting portion of the extending portion in a plan view seen in the thickness direction, a marker portion configured to be visually identified, and
the adhesive sheet is adhered to the biological surface in a state in which an insertion portion of a medical tube member inserted into a living body from the biological surface, the insertion portion being located on the same surface as the biological surface, is aligned with the marker portion.

10. The adhering method according to Claim 9, wherein
the marker portion includes a region marker that defines a predetermined region in the plan view seen in the thickness direction, and
the adhesive sheet is adhered to the biological surface in a state in which the whole insertion portion of the medical tube member inserted into the living body from the biological surface, the whole insertion portion being located on the same surface as the biological surface, is aligned with the region marker.

11. The adhering method according to Claim 10, wherein
the marker portion includes a position marker indicating a predetermined reference position in the predetermined region defined by the region marker in the plan view seen in the thickness direction, and
the adhesive sheet is adhered to the biological surface in a state in which the whole insertion portion of the medical tube member inserted into the living body from the biological surface, the whole insertion portion being located on the same surface as the biological surface, is aligned within the region marker, and a state in which a part of the insertion portion is aligned with the position marker.
